# EUROPEAN PATENT SPECIFICATION

(11) **EP 3 695 820 B1**
(45) Date of publication and mention of the grant of the patent: **09.06.2021**
(21) Application number: 17934393.4
(22) Date of filing: 14.12.2017
(51) Int. Cl.: A61F 13/15, B29C 65/08, B29L 31/48, B29C 65/78

(54) **ULTRASONIC SEALING METHOD AND ULTRASONIC SEALING DEVICE**
ULTRASCHALLVERSIEGELVERFAHREN UND ULTRASCHALLVERSIEGELVORRICHTUNG
PROCÉDÉ DE SOUDAGE PAR ULTRASONS ET DISPOSITIF DE SOUDAGE PAR ULTRASONS

(30) Priority: 13.12.2017 JP 2017238624
(43) Date of publication of application: 19.08.2020
(73) Proprietor: Unicharm Corporation, Shikokuchuo-shi, Ehime 799-0111 (JP)
(72) Inventor: NINOMIYA, Akihide, Kanonji-shi, Kagawa7691602 (JP); YAMAMOTO, Hiroki, Kanonji-shi, Kagawa7691602 (JP)
(74) Representative: Staeger & Sperling Partnerschaftsgesellschaft mbB
(86) International application number: PCT/JP2017/044922
(87) International publication number: WO 2019/116505

(56) References cited:
- EP-A1- 2 351 545
- WO-A1-2013/027390
- JP-A- 2002 355 270
- JP-A- 2005 205 026
- JP-A- 2005 212 149
- JP-A- 2014 524 265

## Description

### [Technical Field]

The present invention relates to a method and a device for ultrasonic-sealing.

### [Background Art]

There is a well-known method for ultrasonic-sealing in which seal portions are formed at intervals in a direction of transporting a continuous web for an absorbent article by repeatedly executing ultrasonic-sealing processing on the transported continuous web.

This ultrasonic-sealing processing includes: a clamping step in which the continuous web is clamped between an ultrasonic horn and an anvil; a seal-portion forming step in which seal portions are formed in the continuous web by vibration of the ultrasonic horn due to receiving ultrasonic waves emitted by an ultrasonic wave generator; and an unclamping step in which the continuous web is unclamped.

### [Citation List]

### [Patent Literature]

[PTL 1] Japanese Patent Application Publication No. 2002-355270 Z • Further prior art is provided by documents WO 2013/027390 A1 and EP 2351545 A1.

WO 2013/027390 A1 and EP 2351545 A1 disclose a method for ultrasonic-sealing in which a plurality of seal portions are formed in a continuous web at an interval in a direction of transporting the continuous web, the forming being performed by repeatedly executing ultrasonic-sealing processing on a continuous web that is being transported, the continuous web being for an absorbent article, the ultrasonic-sealing processing comprising: a clamping step of clamping the continuous web with an ultrasonic horn and an anvil; a seal-portion forming step of forming the seal portions in the continuous web by vibration of the ultrasonic horn due to receiving an ultrasonic wave generated by an ultrasonic wave generator; and an unclamping step of unclamping the continuous web after the ultrasonic wave generator has stopped generating the ultrasonic wave.

WO 2013/027390 A1 and EP 2351545 A1 also disclose a device for ultrasonic-sealing that forms a plurality of seal portions in a continuous web that is for an absorbent article, the device for ultrasonic-sealing comprising: a transporting device configured to transport the continuous web; an ultrasonic wave generator configured to generate an ultrasonic wave; an ultrasonic horn configured to form the seal portion in the continuous web that is being transported by the transporting device, by vibrating due to receiving the ultrasonic wave generated by the ultrasonic wave generator; and an anvil configured to clamp the continuous web together with the ultrasonic horn, the device being configured in that the ultrasonic horn forms the plurality of seal portions at an interval in a direction of transporting the continuous web by repeatedly executing ultrasonic-sealing processing, the ultrasonic-sealing processing being for forming a seal portion in the continuous web by vibrating while clamping the continuous web together with the anvil, the ultrasonic horn and the anvil unclamping the continuous web after the ultrasonic wave generator has stopped generating the ultrasonic wave.

### [Summary of Invention]

### [Technical Problem]

Even after the ultrasonic wave generator stops generating ultrasonic waves, residual vibration remains as noise in the ultrasonic horn, and in conventional technology, no consideration whatsoever has been given to the existence of such residual vibration when determining the timing for unclamping the continuous web. Accordingly, unclamping has been performed while residual vibration remains after the ultrasonic wave generator has stopped generating ultrasonic waves.

Residual vibration dissipates more slowly when the ultrasonic horn and the anvil are not in a clamped state (are in an unclamped state) than when they are in a clamped state, and therefore a phenomenon has occurred in which residual vibration continues for a relatively extended time after unclamping.

The present invention was achieved in light of conventional problems such as that described above, and an aspect of the present invention is to swiftly dissipate residual vibration.

### [Solution to Problem]

Amain aspect of the present invention for achieving the above-described aspect is an method for ultrasonic-sealing in which a plurality of seal portions are formed in a continuous web at an interval in a direction of transporting the continuous web,
the forming being performed by repeatedly executing ultrasonic-sealing processing on a continuous web that is being transported,
the continuous web being for an absorbent article,
the ultrasonic-sealing processing including:
a clamping step of clamping the continuous web with an ultrasonic horn and an anvil;
a seal-portion forming step of forming the seal portions in the continuous web by vibration of the ultrasonic horn due to receiving an ultrasonic wave generated by an ultrasonic wave generator; and
an unclamping step of unclamping the continuous web after the ultrasonic wave generator has stopped generating the ultrasonic wave and furthermore residual vibration remaining in the ultrasonic horn has stopped.

Features of the present invention other than the above will become clear by reading the description of the present specification with reference to the accompanying drawings.

### [Advantageous Effects of Invention]

According to the present invention, residual vibration can be swiftly dissipated.

### [Brief Description of the Drawings]

FIG. 1 includes an upper diagram that is a perspective view of an unfolded state of a continuous web 30, and a lower diagram that is a perspective view of the continuous web 30 while being transported to a sealing device 1.
FIG. 2 is a cross-sectional view taken along IX-IX in the lower diagram in FIG. 1, and shows a state where the continuous web 30 is clamped between ultrasonic horns 8 and anvils 14.
FIG. 3 is a cross-sectional view taken along X-X in the lower diagram in FIG. 1 and FIG. 2, and shows a state where the continuous web 30 is clamped between ultrasonic horns 8 and anvils 14.
FIG. 4 is a cross-sectional view taken along I-I in FIG. 9, and shows the sealing device 1 according to an embodiment.
FIG. 5 is a diagram showing a rotating drum 5 of the sealing device 1 and the internal structure thereof.
FIG. 6 is an illustrative diagram for illustrating operations of a swing drive portion.
FIG. 7 is a schematic view of a state in which a sealing unit 10 is clamping the continuous web 30.
FIG. 8 is an enlarged view of the internal structure of a holding portion 52.
FIG. 9 is an illustrative view of operation states of the sealing device 1.
FIG. 10 is a diagram showing the relationship between vibration periods of the ultrasonic horn 8 and a clamp period of the continuous web 30 in ultrasonic-sealing processing.

### [Description of Embodiments]

At least the following matters will become clear with the description of this specification and the attached drawings.

An method for ultrasonic-sealing in which a plurality of seal portions are formed in a continuous web at an interval in a direction of transporting the continuous web,
the forming being performed by repeatedly executing ultrasonic-sealing processing on a continuous web that is being transported,
the continuous web being for an absorbent article,
the ultrasonic-sealing processing including:
a clamping step of clamping the continuous web with an ultrasonic horn and an anvil;
a seal-portion forming step of forming the seal portions in the continuous web by vibration of the ultrasonic horn due to receiving an ultrasonic wave generated by an ultrasonic wave generator; and
an unclamping step of unclamping the continuous web after the ultrasonic wave generator has stopped generating the ultrasonic wave and furthermore residual vibration remaining in the ultrasonic horn has stopped.

According to this method for ultrasonic-sealing, it is possible to swiftly dissipate residual vibration.

In such a method for ultrasonic-sealing, it is desirable that
a period of time from when the residual vibration stops until when the continuous web is unclamped
is longer than
a period of time from when the ultrasonic wave generator stops generating the ultrasonic wave until when the residual vibration stops.

According to this method for ultrasonic-sealing, sufficient time can be provided between when residual vibration stops and when the continuous web is unclamped, and even if the length (time) of a residual vibration period varies, it is possible to reliably unclamp the continuous web after residual vibration has dissipated.

In such a method for ultrasonic-sealing, it is desirable that
a period of time from when the residual vibration stops until when the continuous web is unclamped
is longer than
a period of time from when the ultrasonic horn and the anvil start to clamp the continuous web until when the ultrasonic wave generator starts to generate the ultrasonic wave.

According to this method for ultrasonic-sealing, sufficient time can be provided between when residual vibration stops and when the continuous web is unclamped, and even if the length (time) of a residual vibration period varies, it is possible to reliably unclamp the continuous web after residual vibration has dissipated.

In such a method for ultrasonic-sealing, it is desirable that
a period of time from when the residual vibration stops until when the continuous web is unclamped
is shorter than
a period of time from when the ultrasonic wave generator starts generating the ultrasonic wave until when the ultrasonic wave generator stops generating the ultrasonic wave.

According to this method for ultrasonic-sealing, it is possible to swiftly dissipate residual vibration.

In such a method for ultrasonic-sealing, it is desirable that
in the clamping step, the continuous web is clamped in a state where the ultrasonic horn is not vibrating.

According to this method for ultrasonic-sealing, it is possible to suppress unintended vibration, and vibration of the ultrasonic horn can be started swiftly.

In such a method for ultrasonic-sealing, it is desirable that
in the seal-portion forming step, after the ultrasonic wave generator starts to generate the ultrasonic wave, the ultrasonic wave generator stops generating the ultrasonic wave when an ultrasonic vibration energy has reached a predetermined amount.

According to this method for ultrasonic-sealing, the ultrasonic wave generator can emit a predetermined amount of ultrasonic vibration energy, making it possible to stably form seal portions.

In such a method for ultrasonic-sealing, it is desirable that
in the clamping step, the continuous web is clamped by a flat surface of the ultrasonic horn and a flat surface of the anvil.

According to this method for ultrasonic-sealing, it is possible to swiftly dissipate residual vibration of the ultrasonic horn.

In such a method for ultrasonic-sealing, it is desirable
that the continuous web is transported by rotation of the rotation member with being wound on an outer circumferential surface of a rotation member,
that a plurality of first members, which are either of the ultrasonic horns and the anvils, are provided on the outer circumferential surface of the rotation member at an equal interval,
that a plurality of second members, which are other ones out of the ultrasonic horns and the anvils, are provided on the rotation member in correspondence with the first members,
that each of the plurality of second members can move toward and away from the corresponding first member on an opposite side of the continuous web,
that when each of the ultrasonic horns and the corresponding anvil reaches a predetermined first angular position while rotating along with rotation of the rotation member,
the second member comes into contact with the corresponding first member and clamps the continuous web,
that when each of the ultrasonic horns and the corresponding anvil reaches a predetermined second angular position while rotating along with rotation of the rotation member,
the second member moves away from the corresponding first member and unclamps the continuous web, and
that when each of the ultrasonic horns and the corresponding anvil reaches a predetermined third angular position while rotating along with rotation of the rotation member,
the ultrasonic wave generator starts to generate the ultrasonic wave.

According to this method for ultrasonic-sealing, it is possible to reliably generate the ultrasonic wave at a desired timing regardless of the rotation speed of the rotation member.

In such a method for ultrasonic-sealing, it is desirable that letting θ be a difference between a third angle and a second angle, and T be a period of time from when the ultrasonic wave generator starts to generate the ultrasonic wave until when the residual vibration stops,
the continuous web is transported by rotation of the rotation member at an angular velocity less than or equal to θ/T.

According to this method for ultrasonic-sealing, it is possible to swiftly dissipate residual vibration of the ultrasonic horn.

In such a method for ultrasonic-sealing, it is desirable that letting a low speed mode be a mode in which the continuous web is transported by rotation of the rotation member at a first speed, and
letting a high speed mode be a mode in which the continuous web is transported by rotation of the rotation member at a second speed that is higher than the first speed,
concerning a period of time from when the residual vibration stops until when the continuous web is unclamped,
the period of time in a case where the plurality of seal portions have been formed in the low speed mode,
is longer than
the period of time in a case where the plurality of seal portions have been formed in the high speed mode.

According to this method for ultrasonic-sealing, the length of time from when residual vibration stops until when the continuous web is unclamped can be adjusted by selecting an operation mode (selecting the transporting speed of the continuous web).

In such a method for ultrasonic-sealing, it is desirable that
each of the plurality of second members moves toward and away from the corresponding first member by swinging.

According to this method for ultrasonic-sealing, it is possible to realize a clamping mechanism that has a simple structure and is reliable.

In such a method for ultrasonic-sealing, it is desirable
that the method for ultrasonic-sealing further comprises a frequency adjustment processing of adjusting a frequency of the ultrasonic wave that the ultrasonic wave generator is to generate for the ultrasonic horn, and
that the frequency adjustment processing is executed at a time that is between
when the ultrasonic horn and the anvil have unclamped the continuous web in the ultrasonic-sealing processing and
when the ultrasonic wave generator starts to generate the ultrasonic wave for the ultrasonic horn for a next instance of the ultrasonic-sealing processing to be performed by the ultrasonic horn.

According to this method for ultrasonic-sealing, the frequency adjustment processing is executed in the state where residual vibration has stopped. Frequency adjustment can therefore be performed appropriately.

An device for ultrasonic-sealing that forms a plurality of seal portions in a continuous web that is for an absorbent article, the device for ultrasonic-sealing including:
a transporting device configured to transport the continuous web;
an ultrasonic wave generator configured to generate an ultrasonic wave;
an ultrasonic horn configured to form the seal portion in the continuous web that is being transported by the transporting device, by vibrating due to receiving the ultrasonic wave generated by the ultrasonic wave generator; and
an anvil configured to clamp the continuous web together with the ultrasonic horn,
the ultrasonic horn forming the plurality of seal portions at an interval in a direction of transporting the continuous web by repeatedly executing ultrasonic-sealing processing,
   the ultrasonic-sealing processing being for forming a seal portion in the continuous web by vibrating while clamping the continuous web together with the anvil,
the ultrasonic horn and the anvil unclamping the continuous web after the ultrasonic wave generator has stopped generating the ultrasonic wave and furthermore residual vibration remaining in the ultrasonic horn has stopped.

The above device for ultrasonic-sealing achieves operations and effects similar to those of the method for ultrasonic-sealing described above.

### Method for ultrasonic-sealing of present embodiment

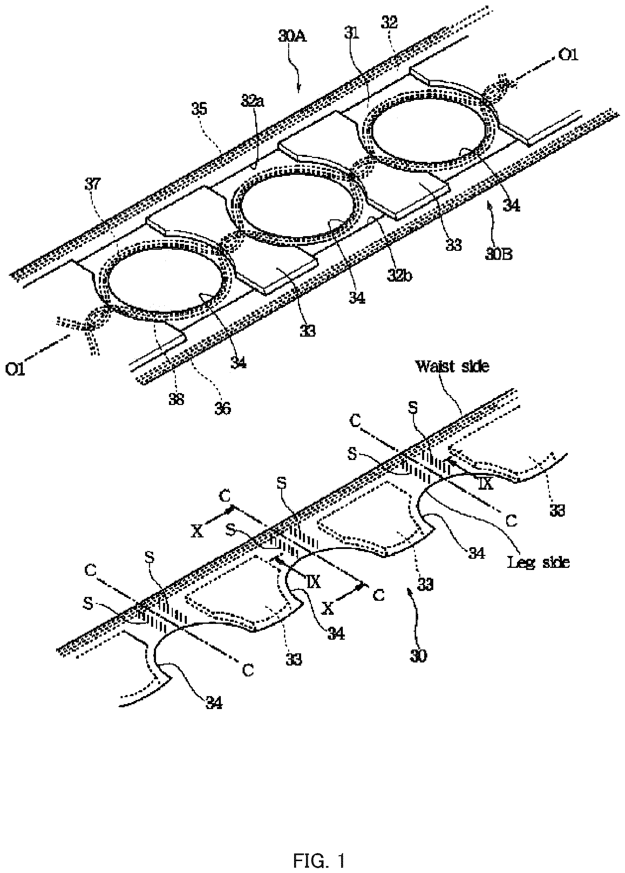

An method for ultrasonic-sealing of the present embodiment is used with an device for ultrasonic-sealing (hereinafter called a sealing device 1) . As shown in the lower diagram in FIG. 1, the sealing device 1 is an device for forming multiple seal portions S at intervals in the direction of transporting a continuous web 30 for an absorbent article (the continuous web 30 is a stack of absorbent article base sheets), which is transported along a continuous production line of the absorbent articles, by repeatedly executing ultrasonic-sealing processing on the continuous web 30.

### Continuous web 30 for absorbent article

First, the continuous web 30 for an absorbent article of the present embodiment will be described by way of example of a pull-on disposable diaper.

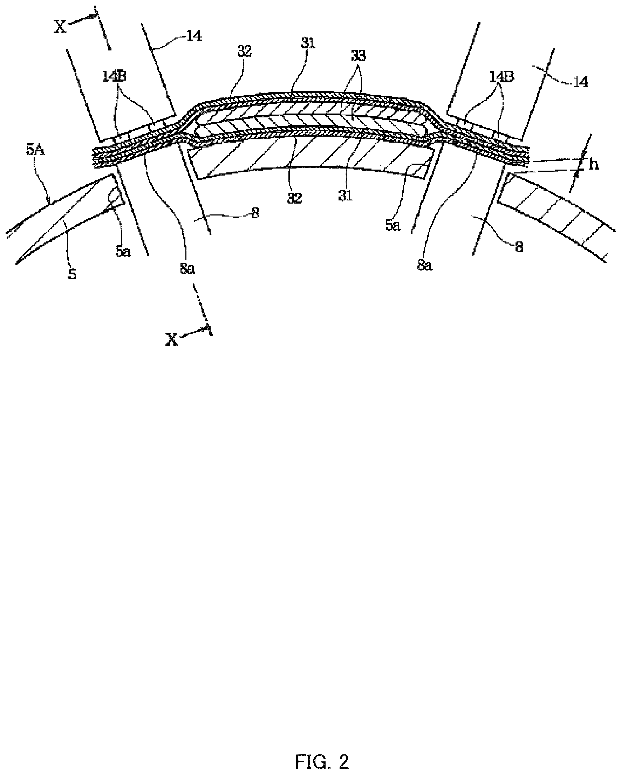

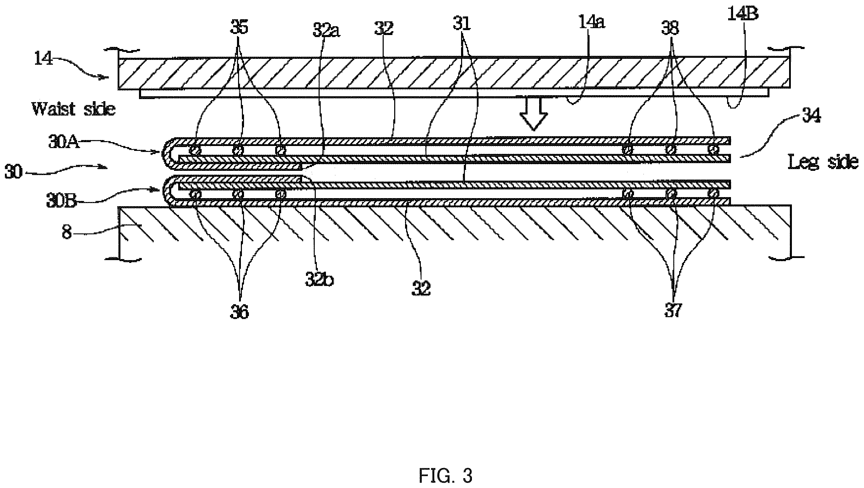

As shown in the lower diagram in FIG. 1, the continuous web 30 of the present embodiment is transported to the sealing device 1 in a folded state. FIG. 2 is cross-sectional view taken along IX-IX in the lower diagram in FIG. 1, and shows a state in which the continuous web 30 is clamped between ultrasonic horns 8 and anvils 14 and seal portions S are being formed in the continuous web 30, and FIG. 3 is a cross-sectional view taken along X-X in the lower diagram in FIG. 1 and FIG. 2.

The lower diagram in FIG. 1 shows a state in which the ultrasonic horns 8 and the anvils 14 of the sealing device 1 have formed seal portions S in the continuous web 30. After the seal portions S have been formed, the continuous web 30 is cut along cut lines C-C between adjacent seal portions S, thus producing pull-on disposable diapers, which are the absorbent article of the present embodiment. Also, the upper diagram in FIG. 1 shows a state in which the continuous web 30 has been unfolded immediately before being transported into the sealing device 1.

As shown in the upper diagram in FIG. 1, when the continuous web 30 is an unfolded belt-like body, a first sheet 32 is located on the underside in the diagram, and a second sheet 31 is stacked thereon. The first sheet 32 is wider than the second sheet 31, and on one side 30A in the upper diagram in FIG. 1, a side edge 32a of the first sheet 32 is folded over onto the second sheet 31. Similarly, on an other side 30B as well, a side edge 32b of the first sheet 32 is folded over onto the second sheet 31. This folded state is shown in FIG. 3.

As indicated by "waist side" in FIG. 3, on the one side 30A of the belt-like body, multiple first waist bands 35 are sandwiched between the first sheet 32 and the second sheet 31. Also, on the other side 30B of the belt-like body, multiple second waist bands 36 are sandwiched between the first sheet 32 and the second sheet 31. The first waist bands 35 and the second waist bands 36 are respectively arranged parallel to each other and extend as straight lines in the direction of transporting the belt-like body.

Also, as indicated by "leg side" in FIG. 3, multiple first leg bands 37 and second leg bands 38 are provided between the first sheet 32 and the second sheet 31. As shown in the upper diagram in FIG. 1, the first leg bands 37 and the second leg bands 38 extend along the direction of transporting the belt-like body while curving in a wave-like shape. Leg holes 34, which form leg insertion portions in the underpants-shaped state, are formed in the regions enclosed within the first leg bands 37 and the second leg bands 38.

The first waist bands 35, the second waist bands 36, the first leg bands 37, and the second leg bands 38 are sandwiched between the first sheet 32 and the second sheet 31 in a state of being stretched by a predetermined factor in the direction of transporting the belt-like body. Also, the first sheet 32 and the second sheet 31 are adhered to the first waist bands 35, the second waist bands 36, the first leg bands 37, and the second leg bands 38, which are sandwiched therebetween, using a hot-melt adhesive or the like.

The first sheet 32 and the second sheet 31 are breathable while also blocking the passage of liquids, and can also be fused together through heat. For example, they can be made of spunbond nonwoven fabric or meltblown nonwoven fabric formed by thermoplastic synthetic fiber, or a laminated body including layers of such nonwoven fabric. Also, a configuration is possible in which either the first sheet 32 or the second sheet 31 is made of nonwoven fabric, and the other one is made of breathable plastic film.

The first waist bands 35, the second waist bands 36, the first leg bands 37, and the second leg bands 38 are elastic stretching members made of rubber or synthetic rubber strings or bands, for example.

A liquid-absorbent body 33 is disposed between adjacent leg holes 34 on the upper surface of the second sheet 31. The liquid-absorbent body 33 is hourglass-shaped or rectangular, and multiple liquid-absorbent bodies 33 are arranged at constant intervals in the direction of transporting the belt-like body. The liquid-absorbent body 33 includes a pulverized pulp, a mixture of a pulverized pulp and a liquid-absorbent polymer (SAP), a stacked body including layers of hydrophilic nonwoven fabric, an air-laid pulp, or the like. These absorbent materials are wrapped in a liquid-permeable top sheet. Each liquid-absorbent body 33 is adhered to the upper surface of the second sheet 31 using a hot-melt adhesive or the like.

The top sheet is formed by spun-lace nonwoven fabric, air-through nonwoven fabric, a plastic film provided with liquid passage holes, or the like.

The belt-like body shown in the upper diagram in FIG. 1 is folded one time onto itself at a center line 01-01 that extends in the longitudinal direction, thus obtaining the continuous web 30 shown in the lower diagram in FIG. 1. As previously described, the continuous web 30 is transported to the sealing device 1 in the form shown in the lower diagram in FIG. 1. Upon the continuous web 30 being transported to the sealing device 1, the first sheet 32 and the second sheet 31, which are overlaid on each other, are clamped and sealed between the ultrasonic horns 8 and the anvils 14 of the sealing device 1 at locations between adjacent liquid-absorbent bodies 33.

Also, in the present embodiment, as shown in FIG. 3, in intermediate portions of the continuous web 30 that are sandwiched between the ultrasonic horns 8 and the anvils 14, four sheets altogether, specifically two portions of the first sheet 32 and two portions of the second sheet 31, are overlaid on each other. The thickness of the continuous web 30 is the smallest in the intermediate portions. On the waist side, in addition to the four sheets, the two side edges of the first sheet 32 are folded back, and the first waist bands 35 and the second waist bands 36 are also sandwiched between the sheets, thus forming the thickest portions. On the leg side corresponding to the leg hole 34 side, the four sheets and the first leg bands 37 and the second leg bands 38 arranged therebetween are provided, and the thickness on the leg side is higher than in the intermediate portions, but lower than on the waist side.

The first sheet 32 and the second sheet 31 are formed from a heat-fusing material, and therefore when heat is generated therein by vibration applied by the ultrasonic horn 8, the sheets become fused together in accordance with a micro protrusion pattern that is formed on an anvil facing surface 14a of the anvil 14, thus forming seal portions S.

In the example shown in the lower diagram in FIG. 1, the pattern of the seal portions S formed by the micro protrusion pattern is a column of thin seal lines. After the seal portions S have been formed by the sealing device 1, the continuous web 30 is cut along cut lines C-C located between adjacent seal portions S, thus forming pull-on disposable diapers corresponding to the absorbent article.

Note that although the manufacturing of a pull-on disposable diaper has been described above as an example of the manufacturing of an absorbent article, the absorbent article manufactured by the sealing device 1 of the present invention may be a sanitary napkin, a panty liner, or the like.

### Sealing device 1

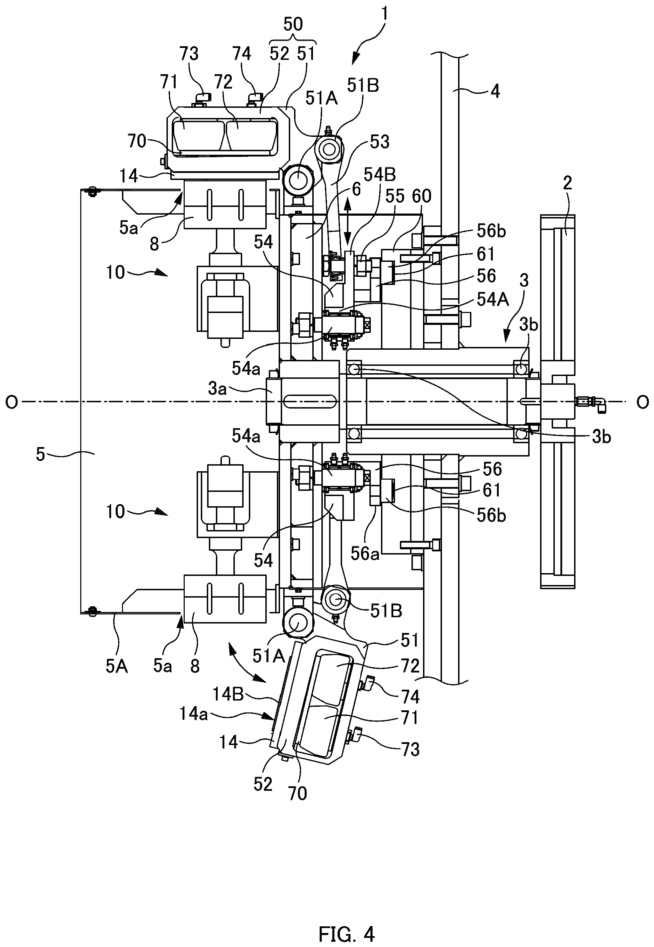

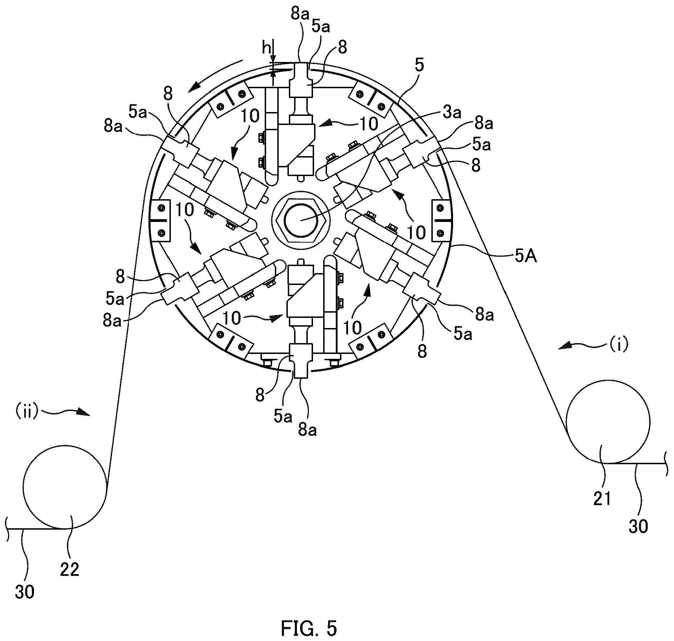

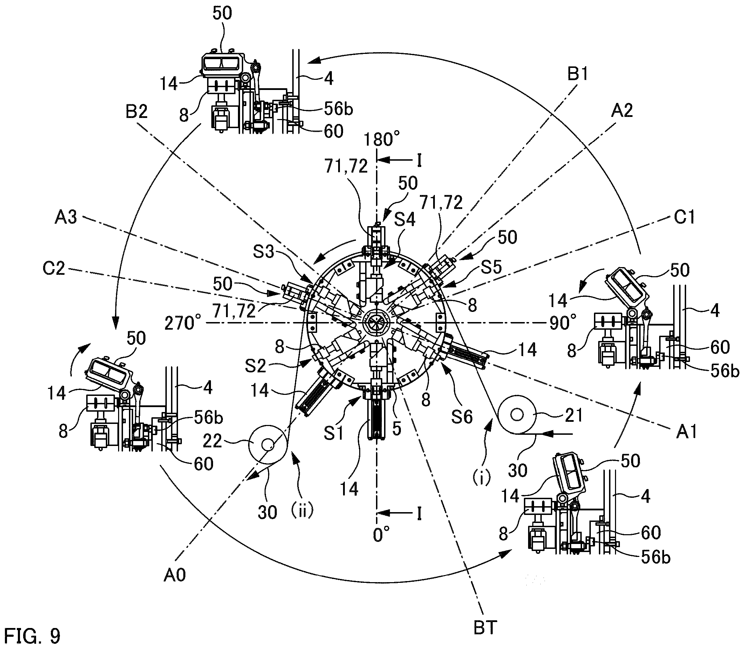

Next, the sealing device 1 will be described. FIG. 4 is a cross-sectional view of the sealing device 1 according to the present embodiment (a cross-section along line I-I in FIG. 9). FIG. 5 is a diagram showing a rotating drum 5 of the sealing device 1 and the internal structure thereof.

The sealing device 1 includes a rotation-member drive portion, a rotation member (corresponding to a transporting device), sealing units 10, ultrasonic wave generators 9, swinging support members 50, and a swing drive portion.

As shown in FIG. 4, the rotation-member drive portion includes a rotation shaft 3a, a bearing portion 3, ball bearings 3b, a timing wheel 2, a toothed belt (not shown), and a motor (not shown). Also, the bearing portion 3 is provided on a fixed table 4, which is a fixed portion, and the rotation shaft 3a is rotatably supported by the ball bearings 3b that are held in the bearing portion 3. In FIG. 4, the center line of the rotation shaft 3a is indicated by a rotation center line O-O.

The timing wheel 2, which has teeth on its outer circumferential surface, is fixed to the base end portion of the rotation shaft 3a on the right side in FIG. 4, and the toothed belt is hung around the timing wheel 2. When a drive source that has a motor applies motive power to the timing wheel 2 via the toothed belt, the rotation shaft 3a continuously rotates in the counter-clockwise direction as seen from the left side in FIG. 4.

The rotation member is for transporting the continuous web 30, and includes a rotation base 6 and the rotating drum 5 as shown in FIG. 4. The rotation member is fixed to the rotation shaft 3a, and rotates due to the rotation-member drive portion. Specifically, the rotation base 6 is fixed to the rotation shaft 3a, the rotating drum 5 is fixed to the rotation base 6, and these members rotate along with the rotation shaft 3a. The rotation base 6 is parallel with and opposes the fixed table 4 across a gap for ensuring space for provision of the swinging support members 50 and the swing drive portion.

As shown in FIGS. 4 and 5, an outer circumferential surface 5A of the rotating drum 5 is provided with multiple windows 5a that are rectangular and extend parallel with the rotation center line O-O. The windows 5a are formed at an even pitch in the circumferential direction of the rotating drum 5, and in the present embodiment, six windows 5a are provided at an arrangement angle of 60 degrees about the rotation center line O-O.

The sealing units 10 each include an ultrasonic horn 8, a booster 7b, a converter 7a, and an anvil 14, and are provided on the rotation member.

Accordingly, the sealing units 10 rotate along with the rotation base 6 and the rotating drum 5. In the present embodiment, as shown in FIG. 5, multiple (six) sealing units 10 are provided at an equiangular arrangement angle of 60 degrees along the circumferential direction of the rotation member. Hereinafter, the sealing units 10 will be called a sealing unit S1, a sealing unit S2, a sealing unit S3, a sealing unit S4, a sealing unit S5, and a sealing unit S6, in order along the circumferential direction.

As shown in FIG. 4, the ultrasonic horn 8 is fixed to the rotation base 6 at a location inside the rotating drum 5. The ultrasonic horns 8 and the devices connected to the ultrasonic horns 8 are arranged in a radiating manner about the rotation center line O-O, and the arrangement angle thereof matches the arrangement angle of the windows 5a. Also, as shown in FIG. 5, the ultrasonic horns 8 each protrude outward from the corresponding window 5a of the rotating drum 5 to a projecting height h, a horn facing surface 8a at the leading end of the ultrasonic horn 8 faces outward in the normal direction (radial direction) from the rotation center line O-O, and the horn facing surface 8a is parallel with the rotation center line O-O.

The anvils 14 are fixed to support portions 70 of holding portions 52 of the swinging support members 50, and move toward and away from the horn facing surfaces 8a as the swinging support members 50 swing. In other words, the anvils 14 each move to a position in contact with the corresponding ultrasonic horn 8, and move to a distant position rotated about 90 degrees.

Note that the sealing units 10 will be described in further detail later.

As shown in FIG. 4, each of the swinging support members 50 is constituted by a swing portion 51 and a holding portion 52, and the swing portion 51 and the holding portion 52 are fixed. The anvils 14 are fixed to the support portions 70, which are provided on the holding portions 52. The swinging support members 50 are provided on the rotation member. Accordingly, the swinging support members 50 rotate along with the rotation base 6 and the rotating drum 5. Note that the holding portions 52 will be described in further detail later.

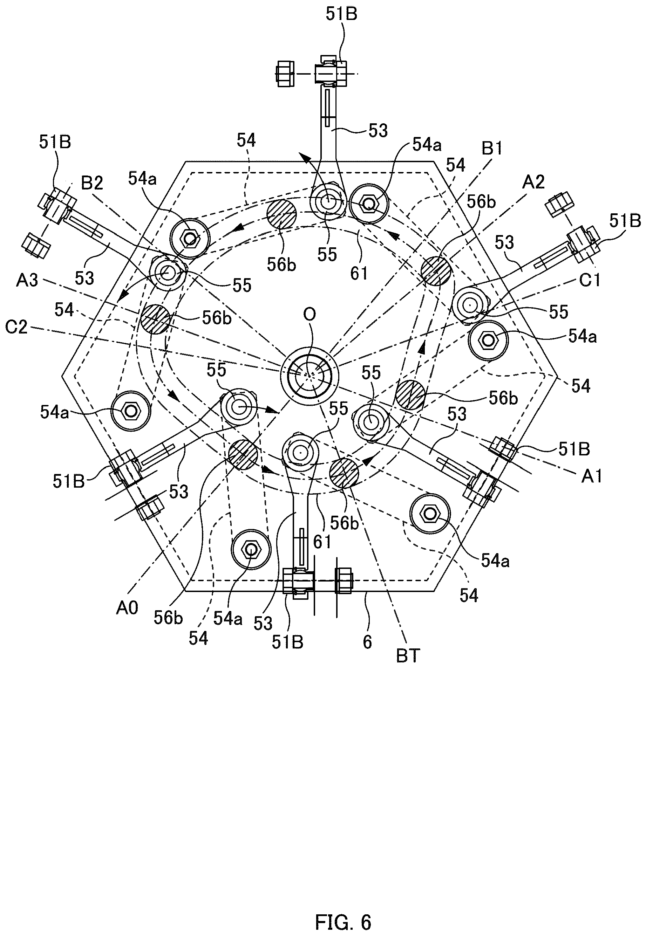

FIG. 6 is an illustrative diagram for illustrating swinging operations of the swinging support members 50. As shown in FIG. 6, the rotation base 6 is has a regular hexagonal front surface. The six swinging support members 50 are provided at intervals of 60 degrees at the center portions of the sides of the regular hexagon, and the arrangement angle of the swinging support members 50 matches the arrangement angle of the ultrasonic horns 8.

As shown in FIG. 4, the swing portions 51 are swingably supported to respective sides of the regular hexagon of the rotation base 6 by swing shafts 51A, and the swing shafts 51A are oriented orthogonal to the rotation center line O-O. Accordingly, the swinging support members 50 (holding portions 52) can swing the anvils 14 about the swing shafts 51A to a position radiating away from rotation center line O-O.

As shown in FIG. 4, in the swing drive portion, a cam member 60 that constitutes the swing drive portion is fixed to the upper surface of the fixed table 4 that faces the rotation base 6. The cam member 60 is shaped as a flat plate that has a predetermined thickness, and as shown in FIG. 6, the front surface is provided with a cam groove 61 that serves as a cam track. The cam groove 61 is continuous around the rotation center line O-O. Also, the recession depth direction of the cam groove 61 is parallel with the rotation center line O-O.

As shown in FIGS. 4 and 6, a link mechanism is provided between the cam member 60 and each of the swing portions 51. In the link mechanism, drive links 53 are rotatably supported to the swing portions 51 via first coupling shafts 51B that are oriented orthogonal to the rotation center line O-O.

Base portions 54A of rotation links 54 are rotatably supported to the rear surface of the rotation base 6 by support shafts 54a. The axial directions of the support shafts 54a are parallel with the rotation center line O-O. The leading end portions of the drive links 53 are rotatably coupled to the leading end portions of arm portions 54B of the rotation links 54 via second coupling shafts 55. The axial directions of the second coupling shafts 55 are parallel with the rotation center line O-O.

A drive member 56 is attached to each of the rotation links 54. The drive member 56 has a drive support body 56a that is non-rotatably fixed to an intermediate portion of the rotation link 54, and a follower 56b that is provided on the drive support body 56a. The follower 56b can move inside the cam groove 61. The follower 56b rolls inside the cam groove 61, or slides therein without rolling.

Note that FIG. 6 shows the relative positional relationship that the rotation links 54 and the followers 56b have with the drive links 53, and the drive support bodies 56a are not shown.

As shown in FIG. 6, when the rotation base 6 rotates, the followers 56b move along the cam groove 61. At this time, the distance between each of the followers 56b and the rotation center line O-O changes according to the shape of the cam groove 61. The rotation links 54 rotate in accordance with this change, and the swinging support members 50 also rotate via the drive links 53.

As previously described, the swinging support members 50 rotate due to the cam groove 61, and as a sealing unit 10 moves from an angular position A2 to an angular position A3 shown in FIG. 6, the swinging support member 50 rotates toward the outer circumferential surface 5A of the rotating drum 5 and then reaches a contact position, as shown by the sealing unit 10 at the top in FIG. 4. Also, as the sealing unit 10 moves from an angular position A0 to an angular position A1 shown in FIG. 6, the swinging support member 50 rotates by about 90 degrees toward the fixed table 4 to a retracted position (see FIG. 9), as shown by the sealing unit 10 at the bottom in FIG. 4.

### Sealing units 10 and ultrasonic wave generators 9

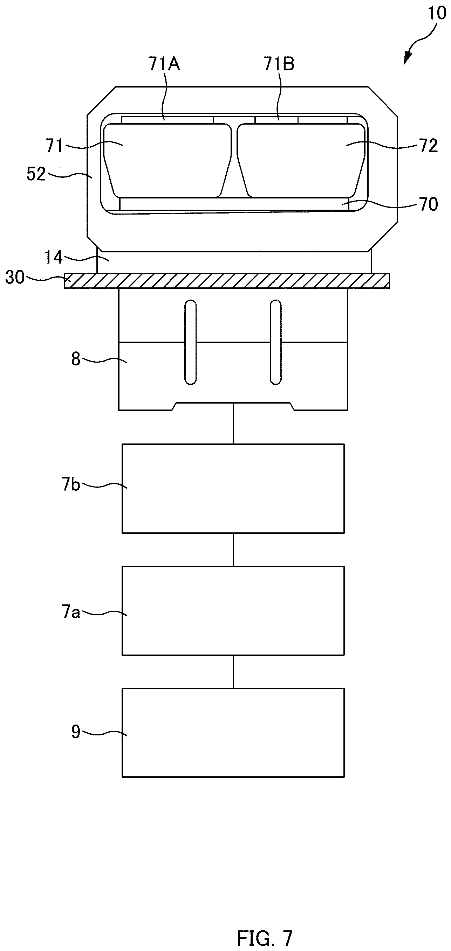

FIG. 7 is a schematic view of a state in which one of the sealing units 10 is clamping the continuous web 30.

In the sealing unit 10, the transported continuous web 30 is clamped between the ultrasonic horn 8 and the anvil 14, an ultrasonic signal emitted (generated) by the ultrasonic wave generator 9 is converted into mechanical ultrasonic vibration by the converter 7a, and the ultrasonic horn 8 receives the mechanical ultrasonic vibration via the booster 7b. The ultrasonic horn 8 vibrates upon receiving the mechanical ultrasonic vibration, and a seal portion S is thus formed in the continuous web 30.

In the present embodiment, a different ultrasonic wave generator 9 emits ultrasonic waves for each of the six sealing units 10. In other words, one ultrasonic wave generator 9 is provided for each of the sealing units 10. Accordingly, as shown in FIG. 7, each sealing unit 10 has one ultrasonic horn 8, one booster 7b, one converter 7a, and one anvil 14, and is connected to one ultrasonic wave generator 9. Note that the ultrasonic wave generators 9 are provided outside of the rotation member and are connected thereto via a slip ring (not shown) . Also, six first members, which are either the ultrasonic horns 8 or the anvils 14 (in the present embodiment, the ultrasonic horns 8), are provided at equal intervals on the outer circumferential surface 5A, and six second members, which are the other ones (in the present embodiment, the anvils 14), are provided on the rotation member in correspondence with the first members (ultrasonic horns 8) and can move toward and away from the first members (ultrasonic horns 8) on the opposite side of the continuous web 30. Also, each second member (anvil 14) moves toward and away from the corresponding first member (ultrasonic horn 8) by swinging.

The ultrasonic wave generator 9 is a device that emits ultrasonic waves (an ultrasound electrical signal) . The frequency of the ultrasonic waves emitted by the ultrasonic wave generator 9 (hereinafter, also called the ultrasonic frequency) is equivalent to the natural frequency of the ultrasonic horn 8 (i.e., the resonance frequency, which in stricter terms is the natural frequency of the ultrasonic stack including the converter 7a and the booster 7b as well, but will be described here as the natural frequency of the ultrasonic horn 8) . In other words, the size (length) of the ultrasonic horn 8 is designed such that the ultrasonic frequency emitted by the ultrasonic wave generator 9 (in the present embodiment, 20kHz) is equivalent to the natural frequency of the ultrasonic horn 8.

The converter 7a is connected to the ultrasonic wave generator 9, and converts the ultrasound electrical signal emitted by the ultrasonic wave generator 9 into mechanical vibration. The converter 7a is provided with a piezoelectric element, and the aforementioned conversion is performed by the piezoelectric element.

The booster 7b is connected to the converter 7a, and amplifies the vibration received from the converter 7a.

The ultrasonic horn 8 is connected to the booster 7b. And, the ultrasonic horn 8 generates frictional heat at the interface between overlaid base materials in the continuous web 30 by vibrating due to receiving ultrasonic waves emitted by the ultrasonic wave generator 9 (ultrasonic waves in the form of mechanical vibration due to the conversion into mechanical vibration performed by the converter 7a), that is to say by applying mechanical energy from vibration to the continuous web 30. Accordingly, the base materials melt and form a seal portion S in the continuous web 30.

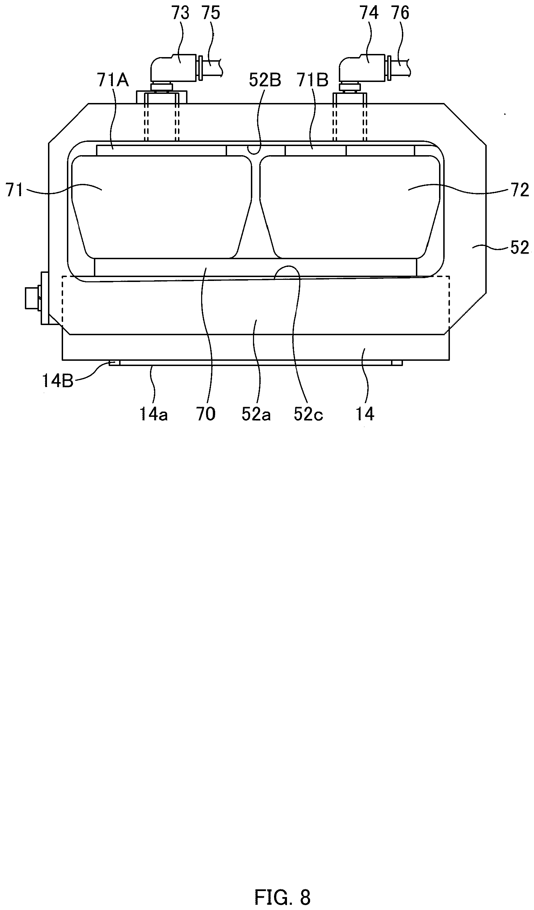

The anvil 14 is a member for performing sealing by clamping the continuous web 30 together with the ultrasonic horn 8. The surface of the anvil 14 is provided with projection portions 14B (see FIG. 8), and the surfaces of the projection portions 14b make up the anvil facing surface 14a that faces the ultrasonic horn 8. The anvil facing surface 14a is a patterned surface set with a pattern corresponding to the seal that is to be formed in the continuous web 30. The anvil 14 is fixed to a support portion 70, which is provided inside the holding portion 52, so that the patterned surface faces outward from the holding portion 52, and the support portion 70 supports a first elastic member 71 and a second elastic member 72 that will be described later.

### Frequency adjusting (tuning) function of ultrasonic wave generator 9

The size (length) of the ultrasonic horn 8 can change due to wear over time and/or expansion or contraction in temperature. If the length of the ultrasonic horn 8 changes, its natural frequency will also change. If the natural frequency changes, a problem occurs in which the ultrasonic frequency emitted by the ultrasonic wave generator 9 no longer matches the natural frequency, and this can cause a problem such as a seal failure in the continuous web 30.

In order to suppress the occurrence of such problems, the ultrasonic wave generator 9 has, as a standard function, a frequency adjusting (tuning) function for adjusting the frequency of the ultrasonic waves that the ultrasonic wave generator 9 applies to the ultrasonic horn 8. When frequency adjustment is performed, the ultrasonic wave generator 9 actually emits ultrasonic waves for frequency adjustment, and detects the ultrasonic frequency that corresponds to the natural frequency of the ultrasonic horn 8. The detected ultrasonic frequency is used when seal-portion formation processing is subsequently performed by the ultrasonic horn 8.

In the present embodiment, the adjusted (detected) frequency is temporarily stored in a storage unit such as a memory provided in the ultrasonic wave generator 9. When seal portion formation is to be subsequently performed, that frequency is read out from the storage unit, the ultrasonic wave generator 9 starts to emit ultrasonic waves in accordance with the ultrasonic frequency.

Note that the ultrasonic wave generator 9 of the present embodiment has, as a standard function and as a different function from the tuning function, a function that is for, when executing seal-portion formation processing, forming a seal portion S while adjusting the ultrasonic frequency in accordance with change thereof, the change being caused by vibration with the continuous web 30 being clamped by the ultrasonic horn 8 and the anvil 14. In other words, even when seal-portion formation processing is performed, the natural frequency can change from time to time, and the ultrasonic wave generator 9 changes the ultrasonic frequency while tracking such change in the natural frequency. Accordingly, in the present embodiment, the ultrasonic frequency acquired by the frequency adjusting function is only used when starting to emit ultrasonic waves.

Also, in the present embodiment, the amplitude of the ultrasonic waves emitted by the ultrasonic wave generator 9 in frequency adjustment is smaller than the amplitude of the ultrasonic waves emitted by the ultrasonic wave generator 9 when forming the seal portion S. The ultrasonic waves emitted by the ultrasonic wave generator 9 in frequency adjustment do not need an amplitude sufficient for melting the continuous web 30, and it is sufficient to be able to detect the ultrasonic frequency that corresponds to the natural frequency of the ultrasonic horn 8. Accordingly, in the present embodiment, the amplitude of the ultrasonic waves emitted by the ultrasonic wave generator 9 in frequency adjustment is about 5% of the amplitude of the ultrasonic waves emitted by the ultrasonic wave generator 9 when forming the seal portion S. This therefore enables suppressing the amount of power that is consumed when the ultrasonic wave generator 9 performs frequency adjustment.

### Holding portion 52

FIG. 8 is an enlarged view of the internal structure of one holding portion 52. The holding portion 52 includes the first elastic member 71 and the second elastic member 72, and the anvil 14 is fixed to the support portion 70 that supports the first elastic member 71 and the second elastic member 72. The first elastic member 71 and the second elastic member 72 are arranged side-by-side in the width direction of the rotating drum 5, that is to say in a direction along the rotation center line O-O. The holding portion 52 swings due to the swing drive portion so as to move the anvil 14 toward or away from the ultrasonic horn 8. The anvil 14 and the ultrasonic horn 8 clamp the continuous web 30 when the holding portion 52 is at a contact position at which the anvil 14 and the ultrasonic horn 8 are in contact with each other.

The first elastic member 71 and the second elastic member 72 are each an air damper or an air spring that includes a bag body (casing) that is flexibly deformable and elastically deformable and made of an elastically deformable material such as rubber or rubber embedded with a reinforcing material. The hollow portion of the bag body receives a supply of air as a fluid such that the interior is set to a predetermined pressure.

As shown in FIG. 8, the attachment sides of the first elastic member 71 and the second elastic member 72 are fixed to an attachment surface 52B of the holding portion 52 via a first fixing plate 71A and a second fixing plate 71B. The support portion 70 is fixed to the pressure application sides of the first elastic member 71 and the second elastic member 72, and the anvil 14 is fixed to the support portion 70. When the anvil 14 is not in contact with the ultrasonic horn 8, the support portion 70 is pressed against an inward surface 52c of a frame portion 52a due to the fluid pressure in the first elastic member 71 and the second elastic member 72.

The first fixing plate 71A and the second fixing plate 71B are respectively provided with a first nozzle 73 and a second nozzle 74, and the first nozzle 73 and the second nozzle 74 are respectively connected to a first air pipe 75 and a second air pipe 76. In other words, the first elastic member 71 and the second elastic member 72 each have an air supply portion, and the internal pressures thereof can be set individually.

As previously described, the continuous web 30 of the present embodiment is the above-described continuous body of pull-on disposable diapers, and as shown in FIG. 3, the continuous web 30 is not flat in the portions where the seal portions S are formed by the ultrasonic horns 8 and the anvils 14. In other words, the thickness is highest on the waist side, the thickness is lower in the intermediate portion, and the thickness is then higher on the leg side. Accordingly, the continuous web 30 that is to be subjected to sealing has different thicknesses in different portions, and the surface has an uneven shape.

Even in the case where the thickness is not even at the locations where the seal portions S are to be formed, and instead is different at different locations as with a disposable diaper, the anvil facing surface 14a can flexibly accommodate changes in the thickness of the continuous web 30 because the anvil 14 is pressed against the ultrasonic horn 8 by the air dampers that are bag-shaped elastic bodies filled with air (a fluid). In other words, due to the pressure inside the first elastic member 71 and the second elastic member 72, the ultrasonic horn 8 and the anvil 14 can substantially evenly press the locations where the seal portions S are to be formed in the continuous web 30. Accordingly, the seal quality can be made uniform for the seal portion S locations.

Also, the pressure inside the bag bodies of the first elastic member 71 and the second elastic member 72 can be controlled by the supply of air from the first nozzle 73 and the second nozzle 74. Accordingly, control for changing the pressure inside the bag bodies can be easily performed in accordance with the material and the structure of the continuous web 30 that is to be subjected to sealing. For this reason, even if the structure of the continuous web 30 is changed, and the sealing pattern of the anvil facing surface 14a is accordingly changed, simply changing the pressure inside the bag bodies makes it possible to complete the setting for that stage, and to always perform sealing with optimal conditions.

### Operations of sealing device 1

Next, operations of the sealing device 1 will be described with reference to FIGS. 1 to 6 and 9. FIG. 9 is an illustrative view of operation states of the sealing device 1.

As shown in FIG. 5, the continuous web 30 is wound on a supply transporting roll 21 and transported to the outer circumferential surface 5A of the rotating drum 5. The continuous web 30 is wound on the outer circumferential surface 5A of the rotating drum 5 (more specifically, the horn facing surfaces 8a of the ultrasonic horns 8 that project from the outer circumferential surface 5A) over an angle of about 180 degrees, and then separates from the rotating drum 5 in a discharge section (ii), is wound around a discharge transporting roll 22, and is drawn to the outside.

The continuous web 30 is continuously fed to the supply section (i) at a constant speed, and in the sealing device 1, rotation motive power is transmitted to the timing wheel 2, and the rotation shaft 3a as well as the rotating drum 5 and the rotation base 6 (which constitute the rotation member) rotate at a constant angular speed in the counter-clockwise direction in FIGS. 5 and 9.

Here, the continuous web 30 comes into contact with the horn facing surfaces 8a of the ultrasonic horns 8 that project from the outer circumferential surface 5A of the rotating drum 5, and the rotating drum 5 rotates in this state. In the present embodiment, the angular speed of the rotation member is set such that the rotation circumferential speed of the horn facing surfaces 8a matches the supply speed of the continuous web 30. Accordingly, on the outer circumferential surface 5A of the rotating drum 5, the horn facing surfaces 8a of the ultrasonic horns 8 and the continuous web 30 move together without sliding relative to each other. In this way, the continuous web 30 is transported due to rotation of the rotation member (rotating drum 5) while the continuous web 30 is wound on the outer circumferential surface 5A of the rotation member (rotating drum 5).

Also, the circumferential arrangement pitch of the ultrasonic horns 8 that project from the outer circumferential surface 5A of the rotating drum 5 matches the arrangement pitch of the liquid-absorbent bodies 33 and the arrangement pitch of the leg holes 34 in the continuous web 30 shown in the lower diagram in FIG. 1. Accordingly, when the continuous web 30 is transported to the outer circumferential surface 5A of the rotating drum 5, as shown in FIG. 2, each liquid-absorbent body 33 is located between a pair of adjacent ultrasonic horns 8, and portions not including the liquid-absorbent body 33 are arranged on the horn facing surfaces 8a of the ultrasonic horns 8.

While the rotation base 6 and the rotating drum 5 rotate in the counter-clockwise direction, the followers 56b provided in the swing drive portion move along the cam groove 61 of the cam member 60 provided on the fixed table 4.

As shown in FIGS. 6 and 9, as each sealing unit 10 (the anvil 14 and the ultrasonic horn 8) moves from the predetermined angular position A0 to the predetermined angular position A1 due to the rotation of the rotation base 6, the corresponding follower 56b moves toward the rotation center line O-O due to the cam groove 61. Accordingly, at this time, as shown by the sealing unit 10 at the bottom in FIG. 4, the corresponding swinging support member 50 rotates about the swing shaft 51A so as to face outward in the radiating direction, and the anvil 14 held by the swinging support member 50 faces outward at an angle of about 90 degrees relative to the rotation center line O-O.

When the sealing unit 10 (the anvil 14 and the ultrasonic horn 8) moves from the angular position A1 to the predetermined angular position A2 (corresponding to a predetermined first angular position), the follower 56b is guided by the cam groove 61 so as to move circumferentially outward. Accordingly, the swinging support member 50 rotates toward the outer circumferential surface 5A of the rotating drum 5, and when the angular position A2 is reached, the anvil 14 and the ultrasonic horn 8 clamp the continuous web 30. In other words, when the ultrasonic horn 8 and the anvil 14 reach the angular position A2 while rotating along with rotation of the rotation member, the anvil 14 comes into contact with the ultrasonic horn 8 and clamps the continuous web (corresponding to a clamping step) .

As shown in FIG. 2, in the region of the continuous web 30 in which the liquid-absorbent body 33 is not provided, the stacked body, which includes the first sheet 32, the second sheet 31, the first waist bands 35, the second waist bands 36, the first leg bands 37, and the second leg bands 38, is compressed between the horn facing surface 8a of the ultrasonic horn 8 and the anvil facing surface 14a of the anvil 14. Here, the horn facing surface 8a and the anvil facing surface 14a are both flat surfaces, and the continuous web 30 is clamped between the two flat surfaces. In other words, in the clamping step, the continuous web 30 is clamped by the flat surface of the ultrasonic horn 8 and the flat surface of the anvil 14. Note that in the clamping step, the continuous web 30 is clamped in the state where the ultrasonic horn 8 is not vibrating.

Note that as shown in FIG. 9, when the sealing unit 10 (the anvil 14 and the ultrasonic horn 8) reaches a predetermined angular position C1 that is along the path from the angular position A1 to the angular position A2, the ultrasonic horn 8 moves from a separated state of being separated from the continuous web 30 to a contact state of being in contact with the continuous web 30.

This clamping state continues until the predetermined angular position A3 (corresponding to a predetermined second angular position) is reached, and during that time (i.e., while the continuous web 30 is clamped by the anvil 14 and the ultrasonic horn 8 from the angular position A2 to the angular position A3), the seal portions S are formed. In other words, the ultrasonic-sealing processing for forming the seal portions S is processing in which the ultrasonic horn 8 and the anvil 14 clamp the continuous web 30, the seal portions S are formed in the continuous web 30 by vibration of the ultrasonic horn 8 due to receiving ultrasonic waves emitted by the ultrasonic wave generator 9, and then the continuous web 30 is unclamped. This ultrasonic-sealing processing is executed in the period of time from the angular position A2 to the angular position A3.

When the sealing unit 10 (the anvil 14 and the ultrasonic horn 8) reaches a predetermined angular position B1 (corresponding to a predetermined third angular position), the ultrasonic wave generator 9 starts to emit ultrasonic waves for the ultrasonic horn 8. In other words, when the ultrasonic horn 8 and the anvil 14 rotate along with rotation of the rotation member and reach the angular position B1, the ultrasonic wave generator 9 starts to generate ultrasonic waves. Note that in the present embodiment, the angular position of the sealing unit 10 can be detected by an encoder, and an electrical signal is used to notify the ultrasonic wave generator 9 that the sealing unit 10 (the anvil 14 and the ultrasonic horn 8) has reached the angular position B1. The ultrasonic horn 8 vibrates upon receiving ultrasonic waves from the ultrasonic wave generator 9, and forms a seal portion S in the continuous web 30 (corresponding to a seal-portion forming step).

After the ultrasonic wave generator 9 starts to emit ultrasonic waves, when the ultrasonic vibration energy (which corresponds to the electric energy of the ultrasonic wave generator 9) reaches a predetermined amount, the ultrasonic wave generator 9 stops the ultrasonic waves (stops generating ultrasonic waves). In other words, in the seal-portion forming step, after the ultrasonic wave generator 9 starts to generate ultrasonic waves, when the ultrasonic vibration energy reaches a predetermined amount, the ultrasonic wave generator 9 stops generating ultrasonic waves. The predetermined amount is determined in advance in consideration of appropriate formation of the seal portion S. Also, the predetermined amount is determined such that the generation of ultrasonic waves is stopped before the sealing unit 10 (the anvil 14 and the ultrasonic horn 8) reaches the angular position A3 (in the present embodiment, as shown in FIG. 9, the ultrasonic wave generator 9 stops generating ultrasonic waves at the angular position B2).

When the sealing unit 10 (the anvil 14 and the ultrasonic horn 8) reaches the angular position A3, the anvil 14 and the ultrasonic horn 8 unclamp the continuous web 30. Specifically, when the ultrasonic horn 8 and the anvil 14 reach the angular position A3, the anvil 14 moves away from the ultrasonic horn 8, thus unclamping the continuous web (corresponding to an unclamping step) . Then, as the sealing unit 10 (the anvil 14 and the ultrasonic horn 8) moves from the angular position A3 to the angular position A0, the follower 56b is guided toward the rotation center line O-O by the cam groove 61, and the anvil 14 rotates away from the ultrasonic horn 8 and the continuous web 30. When the angular position A0 is reached, as shown by the sealing unit 10 at the bottom in FIG. 4 again, the anvil 14 rotates to a retracted position that is at an angle of about 90 degrees relative to the rotation center line O-O.

Note that as shown in FIG. 9, when the sealing unit 10 (the anvil 14 and the ultrasonic horn 8) reaches a predetermined angular position C2 that is along the path from the angular position A3 to the angular position A0, the ultrasonic horn 8 moves from a contact state of being in contact with the continuous web 30 to a separated state of being separated from the continuous web 30. After separating from the ultrasonic horn 8, the continuous web 30 is transported out of the sealing device 1 by the discharge transporting roll 22, and is cut at the cut lines C-C shown in the lower diagram in FIG. 1, thus producing individual pull-on disposable diapers.

### Operation timing in frequency adjusting (tuning) function

It has already be mentioned that the ultrasonic wave generator 9 is provided with a frequency adjusting (tuning) function, and this frequency adjustment is executed during the operation of the sealing device 1. The timing of execution of the frequency adjustment is indicated by an angular position BT in FIG. 9.

Specifically, in the ultrasonic-sealing processing, the frequency of the ultrasonic waves that the ultrasonic wave generator 9 is to generate for the ultrasonic horn 8 is adjusted (frequency adjustment processing is executed) in the period from when the ultrasonic horn 8 and the anvil 14 have unclamped the continuous web 30 (angular position A3) until when the ultrasonic wave generator 9 starts to generate ultrasonic waves for the ultrasonic horn 8 for the next instance of ultrasonic-sealing processing performed by the ultrasonic horn 8 (angular position B1).

In the present embodiment, when the sealing unit 10 (the anvil 14 and the ultrasonic horn 8) rotates along with rotation of the rotation member and reaches the predetermined angular position BT that is along the path from the angular position A3 to the angular position B1, a frequency adjusting step is executed. In other words, the frequency adjusting step is executed when the ultrasonic horn 8 and the anvil 14 reach a predetermined angular position. In the present embodiment, an electrical signal is used to notify the ultrasonic wave generator 9 that the sealing unit 10 (the anvil 14 and the ultrasonic horn 8) has reached the angular position BT, and upon receiving that notification, the ultrasonic wave generator 9 adjusts the frequency of the ultrasonic waves that are to be emitted for the ultrasonic horn 8.

### Repeated operations of sealing unit 10

A description has been given of operations performed by the sealing unit 10 while the rotation member rotates one time (rotates from the angular position A0 and reaches the angular position A0 again), and among the various operations that are repeated as the rotation member rotates continuously such that one sealing unit 10 (hereinafter, the sealing unit S1 is used as an example in the description) repeatedly reaches the same angular position, the following describes repeated operations that are performed in ultrasonic-sealing processing.

As the sealing unit S1 (the ultrasonic horn 8 and the anvil 14) successively reaches the angular positions A2, B1, B2, and A3, the sealing unit S1 performs operations that correspond to those angular positions and executes ultrasonic-sealing processing. After the rotation member rotates one full time, and the sealing unit S1 against successively reaches the same angular positions A2, B1, B2, and A3, the sealing unit S1 again performs operations that correspond to the same angular positions and executes ultrasonic-sealing processing. In other words, the sealing unit S1 executes ultrasonic-sealing processing at an interval of one time per one full rotation of the rotation member, and repeatedly executes the ultrasonic-sealing processing in accordance with the rotation of the rotation member.

### Relationship between vibration of ultrasonic horn 8 and clamping operations

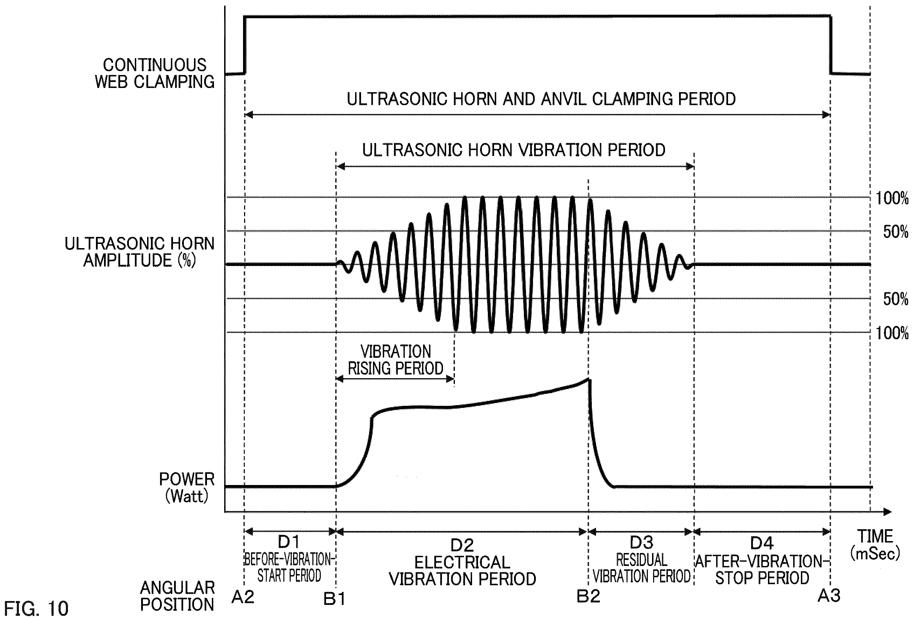

The relationship between operations from the start to the stop of vibration of the ultrasonic horn 8 and from the clamping to the unclamping of the continuous web 30 by the ultrasonic horn 8 and the anvil 14 in the present embodiment will be described below with reference to FIG. 10. FIG. 10 is a diagram showing the relationship between vibration periods of the ultrasonic horn 8 and clamp periods of the continuous web 30 in ultrasonic-sealing processing. The horizontal axis corresponds to time (mSec = 1/1000 seconds) and indicates state change over time for "continuous web clamping", "ultrasonic horn", and "power".

At the top of FIG. 10, "continuous web clamping" indicates the period from clamping to unclamping of the continuous web 30 by the ultrasonic horn 8 and the anvil 14. In other words, this shows the period from the angular position A2 to the angular position A3.

Below "continuous web clamping" in FIG. 10, "ultrasonic horn" indicates the period from the start to the stop of vibration of the ultrasonic horn 8, using the amplitude of the vibration waveform. In other words, when the angular position B1 is reached, the ultrasonic horn 8 receives ultrasonic waves generated by the ultrasonic wave generator 9 and starts to vibrate. The amplitude of the vibration, which was at a still state corresponding to a nil vibration amplitude, moves through a vibration rising period (vibration amplitude increase period) and reaches a predetermined amplitude (100% amplitude shown in FIG. 10). The ultrasonic horn 8 then vibrates with the predetermined amplitude until the angular position B2 is reached.

Then, at the angular position B2, the ultrasonic wave generator 9 stops generating ultrasonic waves, but this stop corresponds to switching off the ultrasonic wave generator 9 so to speak, and merely refers to setting the electrical switch (circuit switch) for the generation of ultrasonic waves by the ultrasonic wave generator 9 to the off state. In other words, the ultrasonic wave generator 9, which is the drive source of the ultrasonic horn 8, merely stops supplying ultrasonic waves to the ultrasonic horn 8. For this reason, mechanical vibration (residual vibration) remains in the ultrasonic horn 8, which is a physical resonating body.

In order to stop mechanical vibration of the ultrasonic horn 8, it is necessary to not only stop the supply of ultrasonic waves from the drive source, but also to quench vibration from the state of mechanical vibration at the predetermined amplitude to the still state of a nil amplitude. In other words, it is necessary to provide a means for switching from the predetermined amplitude state to the still state of a nil amplitude, and to provide time for achieving the still state. For example, in the case of naturally stopping vibration through resting in the air (through vibration attenuation), air resistance or resistance force such as friction force is used as the means, and time is required for the means to achieve the still state.

Accordingly, even when the ultrasonic wave generator 9 stops generating ultrasonic waves, mechanical vibration remains as residual vibration in the ultrasonic horn 8. In other words, as shown in FIG. 10, the vibration period of the ultrasonic horn 8 is the period that starts at the angular position B1, extends beyond the angular position B2, and ends when the residual vibration stops.

Below "ultrasonic horn" in FIG. 10, "power" indicates change in the power (corresponding to electrical power (Watts)) from the start to the stop of the emission of ultrasonic waves by the ultrasonic wave generator 9. In the present embodiment, the ultrasonic wave generator 9 stops generating ultrasonic waves when the ultrasonic vibration energy (corresponding to electric energy (watt-hour)) of ultrasonic waves generated by the ultrasonic wave generator 9 has reached a predetermined amount. In other words, the ultrasonic wave generator 9 starts to generate ultrasonic waves at the angular position B1, and then the ultrasonic wave generator 9 stops generating ultrasonic waves at the angular position B2 at which the integrated value (ultrasonic vibration energy) of the power graph reaches a predetermined value.

Next, considering the vibration period of the ultrasonic horn 8 and the clamp period of the continuous web 30 shown in FIG. 10 to be one continuous period, that one period is divided into multiple periods (see the descriptions under the graph in FIG. 10), and the ultrasonic-sealing processing of the present embodiment will be described below with references to these periods.

First, the period from the angular position A2 to the angular position B1 is a period from when the ultrasonic horn 8 and the anvil 14 start to clamp the continuous web 30 until when vibration of the ultrasonic horn 8 starts. This period will be called a "before-vibration-start period D1".

Next, the period from the angular position B1 to the angular position B2 is a period in which the ultrasonic horn 8, which has started vibrating, continues to vibrate due to receiving an ultrasound electrical signal generated by the ultrasonic wave generator 9. This period will be called an "electrical vibration period D2".

As previously described, when the ultrasonic horn 8 reaches the angular position B2, the ultrasonic wave generator 9 stops generating ultrasonic waves, but even thereafter, mechanical vibration remains as residual vibration in the ultrasonic horn 8. Such residual vibration stops after a delay from when the ultrasonic wave generation stops. In other words, the period from the angular position B2 until residual vibration has stopped is a period in which the ultrasonic horn 8 is mechanically vibrating without a drive source. This period will be called a "residual vibration period D3".

Lastly, the period from when the residual vibration stops until the angular position A3 at which the continuous web 30 becomes unclamped is a period in which the ultrasonic horn 8 is not vibrating. This period will be called an "after-vibration-stop period D4".

In other words, the ultrasonic-sealing processing of the present embodiment can be divided into four periods from the before-vibration-start period D1 to the after-vibration-stop period D4.

In the following, ordinary ultrasonic-sealing processing is divided into periods having the same size as those described above. The ultrasonic-sealing processing of the present embodiment will be described below by describing the differences between the divided periods in ordinary ultrasonic-sealing processing and the periods in the ultrasonic-sealing processing of the present embodiment. Ordinary ultrasonic-sealing processing has a clamping step in which the ultrasonic horn 8 and the anvil 14 clamp the continuous web 30, a seal-portion forming step in which seal portions S are formed in the continuous web 30 by vibration of the ultrasonic horn 8 due to receiving ultrasonic waves emitted by the ultrasonic wave generator 9, and an unclamping step in which the continuous web 30 is unclamped. In other words, ordinary ultrasonic-sealing processing refers to the processing from when the ultrasonic horn 8 and the anvil 14 clamp the continuous web 30 until when the seal portion S is formed and the continuous web 30 is unclamped.

First, consider the processing from when the ultrasonic horn 8 and the anvil 14 start to clamp the continuous web 30 until when the seal portion S is formed to be one period. This period is the same as the before-vibration-start period D1 and the electrical vibration period D2 of the present embodiment.

Then, consider the processing from after the formation of the seal portion S until the continuous web 30 is unclamped to be one period. In this case, this period does not match any of the periods of the present embodiment.

Instead of that period, the present embodiment has a period that includes the residual vibration period D3 and the after-vibration-stop period D4, but in these periods, processing is executed for unclamping the continuous web 30 after residual vibration has been stopped, and therefore this does not correspond to the aforementioned period. In other words, in the processing for that one period, processing is executed for unclamping the continuous web 30 before residual vibration stops (or while ultrasonic vibration continues) . That is to say, the ultrasonic-sealing processing of the present embodiment is different from ordinary ultrasonic-sealing processing in that a period is provided to allow for the residual vibration of the ultrasonic horn 8 to stop after the formation of the seal portion S, and a period is also provided for unclamping the continuous web 30 after the residual vibration has stopped.

In other words, the ultrasonic-sealing processing of the present embodiment includes a clamping step in which the ultrasonic horn 8 and the anvil 14 clamp the continuous web 30, a seal-portion forming step in which seal portions S are formed in the continuous web 30 by vibration of the ultrasonic horn 8 due to receiving ultrasonic waves generated by the ultrasonic wave generator 9, and an unclamping step in which the continuous web 30 is unclamped after the ultrasonic wave generator 9 has stopped generating ultrasonic waves and furthermore residual vibration remaining in the ultrasonic horn 8 has stopped.

The following describes the relationship between the length (time length) of the after-vibration-stop period D4 and the lengths (time lengths) of the sections from the before-vibration-start period D1 to the residual vibration period D3, which are divided as described above. The lengths of the periods in FIG. 10 are representative illustrations of approximate time lengths.

A comparison of the lengths of the after-vibration-stop period D4 and the before-vibration-start period D1 shows that ultrasonic-sealing processing is executed such that the after-vibration-stop period D4 is longer than the before-vibration-start period D1. In other words, the time from when the residual vibration stops until the continuous web 30 is unclamped is longer than the time from when the ultrasonic horn 8 and the anvil 14 start to clamp the continuous web 30 until when the ultrasonic wave generator 9 starts to generate ultrasonic waves. In the present embodiment, the after-vibration-stop period D4 is about 40 to 50 mSec, and the before-vibration-start period D1 is about 30 mSec.

A comparison of the lengths of the after-vibration-stop period D4 and the electrical vibration period D2 shows that, in the present embodiment, ultrasonic-sealing processing is executed such that the after-vibration-stop period D4 is shorter than the electrical vibration period D2. In other words, the time from when the residual vibration stops until when the continuous web 30 is unclamped is shorter than the time from the start to the stop of the generation of ultrasonic waves by the ultrasonic wave generator 9. In the present embodiment, the after-vibration-stop period D4 is about 40 to 50 mSec, and the electrical vibration period D2 is about 85 to 90 mSec.

A comparison of the lengths of the after-vibration-stop period D4 and the residual vibration period D3 shows that, in the present embodiment, ultrasonic-sealing processing is executed such that the after-vibration-stop period D4 is longer than the residual vibration period D3. In other words, the time from when the residual vibration stops until when the continuous web 30 is unclamped is longer than the time from when the ultrasonic wave generator 9 stops generating ultrasonic waves until when the residual vibration stops . In the present embodiment, the after-vibration-stop period D4 is about 40 to 50 mSec, and the residual vibration period D3 is about 30 mSec.

### Relationship between rotation speed of rotation member and operations

Next, the relationship between the rotation speed of the rotation member (rotating drum 5) and various operations will be described in detail. First, the relationship between the rotation speed of the rotation member and the timing when the ultrasonic wave generator 9 starts to generate ultrasonic waves will be described. As previously described, in the present embodiment, when the ultrasonic horn 8 and the anvil 14 rotate along with rotation of the rotation member and reach the angular position B1, the ultrasonic wave generator 9 starts to generate ultrasonic waves. In other words, regardless of the rotation speed of the rotation member, the ultrasonic wave generator 9 starts to generate ultrasonic waves when the sealing unit 10 reaches the predetermined angular position B1.

The following describes how the operations of vibration of the ultrasonic horn 8 and clamping change according to the rotation speed of the rotation member with focus on change in the lengths (time lengths) of the above-described periods, and also describes the rotation speed of the rotation member in the present embodiment.

As previously described, the angular positions A2, A3, and B1 are predetermined angular positions (first angular position to third angular position). For this reason, the lengths of the periods between these angular positions (shown in FIG. 10) changes according to the rotation speed of the rotation member.

In other words, the angle between the angular position A2 and the angular position A3, the angle between the angular position A2 and the angular position B1, and the angle between the angular position B1 and the angular position A3 are angles of a predetermined magnitude, and therefore the time required to traverse them is shorter the higher (faster) the rotation speed (angular velocity) is. That is to say, the total period length of D1 + D2 + D3 + D4, the length of the period D1, and the total period length of D2 + D3 + D4 are shorter the higher the rotation speed of the rotation member is.

In contrast, the length of the electrical vibration period D2 is the period of time required for the ultrasonic vibration energy of the ultrasonic wave generator 9 to reach a predetermined amount, and therefore does not change according to the rotation speed of the rotation member. As previously described, in the present embodiment, the electrical vibration period D2 is about 85 to 90 mSec (note that this length (time) has a slight amount of variation, but is understood to be the aforementioned length based on experience).

Also, the length of the residual vibration period D3 is the amount of time required for vibration to dissipate, and therefore, similarly to the electrical vibration period D2, does not change according to the rotation speed of the rotation member. In other words, as previously described, the length (time) of the residual vibration period D3 is about 30 mSec (note that this length (time) has a slight amount of variation, but is understood to be the aforementioned length based on experience) .

In the present embodiment, the continuous web 30 is transported by rotation of the rotation member at the following rotation speeds (angular velocities).

Letting the difference between the third angle (angular position B1) and the second angle (angular position A3) be an post-vibration clamping angle θ (angle from B1 to A3), and letting the time from when the ultrasonic wave generator 9 starts to generate ultrasonic waves until when residual vibration stops be a vibration time T (the sum time of the electrical vibration period D2 and the residual vibration period D3), the continuous web is transported by rotation of the rotation member at an angular velocity that is less than or equal to "post-vibration clamping angle θ / vibration time T". In the present embodiment, the post-vibration clamping angle θ is about 110 degrees, and the vibration time T is about 120 mSec, and therefore the rotation speed (angular velocity) of the rotation member is less than or equal to about 0.9 degrees/mSec (about 150 rpm) .

The following describes change in the lengths of the periods that occurs due to different operation modes, with use of two operation modes that are different in terms of the transporting speed of the continuous web 30 (corresponding to the rotation speed (rotation circumferential speed) of the rotation member).

These two operation modes are used in the switching of the transporting speed (supply speed) of the continuous web 30. In other words, these operation modes are used in the switching of the processing speed in ultrasonic-sealing processing, and are mainly used in the adjustment of the number of processes in ultrasonic-sealing processing. In the present embodiment, a low speed mode is used as a first speed mode, and a high speed mode is used as a second speed mode having a higher speed than that in the first speed mode.

As previously described the total period length of D2 + D3 + D4 is shorter the higher the transporting speed of the continuous web 30 is, and therefore is shorter in the high speed mode than in the low speed mode. Also, the lengths of the electrical vibration period D2 and the residual vibration period D3 are not dependent on the transporting speed of the continuous web 30, and therefore are the same in both the low speed mode and the high speed mode. Accordingly, among the periods from the electrical vibration period D2 to the after-vibration-stop period D4, it is only the after-vibration-stop period D4 that is different between the low speed mode and the high speed mode.

Specifically, in the case where multiple seal portions S have been formed in the low speed mode in which the continuous web 30 is transported by rotation of the rotation member at the first speed, the time from when the residual vibration stops until when the continuous web 30 is unclamped is longer than in the case where multiple seal portions S have been formed in the high speed mode in which the continuous web 30 is transported by rotation of the rotation member at the second speed, which is higher than the first speed. The length (time) of the after-vibration-stop period D4 changes according to the transporting speed of the continuous web 30 (the rotation speed of the rotation member), and is longer in the low speed mode than in the high speed mode.

### Effectiveness of method for ultrasonic-sealing of present embodiment

As previously described, in the method for ultrasonic-sealing of the present embodiment, the ultrasonic-sealing processing includes a clamping step in which the ultrasonic horn 8 and the anvil 14 clamp the continuous web 30, a seal-portion forming step in which seal portions S are formed in the continuous web 30 by vibration of the ultrasonic horn 8 due to receiving ultrasonic waves generated by the ultrasonic wave generator 9, and an unclamping step in which the continuous web 30 is unclamped after the ultrasonic wave generator 9 has stopped generating ultrasonic waves and furthermore residual vibration remaining in the ultrasonic horn 8 has stopped. This therefore makes it possible to swiftly dissipate residual vibration of the ultrasonic horn 8.

In conventional examples, no consideration whatsoever is given to the existence of residual vibration as noise when determining the timing of unclamping the continuous web 30. Accordingly, unclamping has been performed while residual vibration remains after the ultrasonic wave generator 9 has stopped generating ultrasonic waves.

Residual vibration dissipates more slowly when the ultrasonic horn 8 and the anvil 14 are not in a clamped state (are in an unclamped state) than when they are in a clamped state, and therefore a phenomenon has occurred in which residual vibration continues for a relatively extended time after unclamping.

In contrast, in the ultrasonic-sealing processing of the present embodiment, the continuous web 30 is unclamped after the ultrasonic wave generator 9 has stopped generating ultrasonic waves and furthermore residual vibration remaining in the ultrasonic horn 8 has stopped. In other words, unclamping is performed after the residual vibration has dissipated, and the clamped state, in which residual vibration dissipates more easily, is maintained until the residual vibration has stopped. For this reason, according to the present embodiment, it is possible to swiftly dissipate residual vibration.

Also, in the present embodiment, the time from when the residual vibration stops until when the continuous web 30 is unclamped is longer than the time from when the ultrasonic wave generator 9 stops generating ultrasonic waves until when the residual vibration stops.

In other words, the after-vibration-stop period D4 shown in FIG. 10 is longer than the residual vibration period D3. According to this configuration, sufficient time can be provided between when the residual vibration stops and when the continuous web 30 is unclamped, and even if the length (time) of the residual vibration period D3 varies, it is possible to reliably unclamp the continuous web after residual vibration has dissipated.

Also, in the present embodiment, the time from when the residual vibration stops until the continuous web 30 is unclamped is longer than the time from when the ultrasonic horn 8 and the anvil 14 start to clamp the continuous web 30 until when the ultrasonic wave generator 9 starts to generate ultrasonic waves.

In other words, the after-vibration-stop period D4 shown in FIG. 10 is longer than the before-vibration-start period D1. In the before-vibration-start period D1, the sequence of first clamping the continuous web 30 and then starting the vibration of the ultrasonic horn 8 is very important, and the length (time) of this period may be short. However, if the after-vibration-stop period D4 is set long, it is possible to provide sufficient time between when residual vibration stops until when the continuous web 30 is unclamped, and even if the length (time) of the residual vibration period D3 varies, it is possible to reliably unclamp the continuous web after residual vibration has dissipated.

Also, in the clamping step of the present embodiment, the continuous web 30 is clamped in the state where the ultrasonic horn 8 is not vibrating.

For this reason, the continuous web 30 can be clamped in a state where the ultrasonic horn 8 is not vibrating. In other words, it is possible to suppress unintended vibration, and the vibration of the ultrasonic horn 8 can be swiftly started.

Also, in the clamping step of the present embodiment, the continuous web 30 is clamped by the flat surface of the ultrasonic horn 8 and the flat surface of the anvil 14.

In other words, by clamping the continuous web between the horn facing surface 8a and the anvil facing surface 14a, which are flat surfaces, residual vibration of the ultrasonic horn 8 can be swiftly dissipated.

Also, in the present embodiment, when the ultrasonic horn 8 and the anvil 14 rotate along with rotation of the rotation member and reach the angular position B1, the ultrasonic wave generator 9 starts to generate ultrasonic waves.

For this reason, regardless of whether the rotation of the rotation member is fast or slow, the ultrasonic wave generator 9 starts to generate ultrasonic waves when the ultrasonic horn 8 and the anvil 14 reach the predetermined angular position B1. Accordingly, regardless of the rotation speed of the rotation member, the generation of ultrasonic waves can be reliably started at a desired timing (e.g., can be reliably started in the clamping state after clamping is performed at the angular position A2) .

Also, in the present embodiment, letting the difference between the third angle and the second angle be θ, and letting the time from when the ultrasonic wave generator 9 starts to generate ultrasonic waves until when residual vibration stops be T, the continuous web is transported by rotation of the rotation member at an angular velocity that is less than or equal to θ/T.

Letting the angular velocity (rotation speed) of the rotation member be A, the relationship θ/T ≥ A is satisfied, and if both sides of the expression are multiplied by T, then the relationship 8 ≥ A × T is satisfied. In other words, the "angle (degrees) that the rotation member rotates during the vibration time T", which is the value resulting from "rotation member angular velocity A (deg/s) × vibration time T (s)", is smaller than the post-vibration clamping angle θ (deg). In other words, after the ultrasonic horn 8 has started vibrating (after the third angular position), the continuous web 30 is not unclamped (the second angular position is not reached) before vibration time T has elapsed. That is to say, the continuous web 30 is unclamped (the second angular position is reached) after vibration (residual vibration) of the ultrasonic horn 8 has stopped (after the vibration time T has elapsed), and therefore the residual vibration of the ultrasonic horn 8 can be swiftly dissipated.

Also, in the present embodiment, in the case where multiple seal portions S have been formed in the low speed mode in which the continuous web 30 is transported by rotation of the rotation member at the first speed, the time from when the residual vibration stops until when the continuous web 30 is unclamped is longer than in the case where multiple seal portions S have been formed in the high speed mode in which the continuous web 30 is transported by rotation of the rotation member at the second speed, which is higher than the first speed.

For this reason, the time from when residual vibration stops until when the continuous web is unclamped (i.e., the allowance) can be adjusted by selecting one of the operation modes (selecting the transporting speed of the continuous web 30).

Also, in the present embodiment, the anvil 14 moves toward and away from the ultrasonic horn 8 by swinging.

For this reason, this movement can be realized by merely providing a mechanism for swinging the anvil 14, that is to say, it is possible to realize a clamping mechanism that has a simple structure and is reliable.

Also, in the ultrasonic-sealing processing of the present embodiment, frequency adjustment processing for adjusting the frequency of the ultrasonic waves that the ultrasonic wave generator 9 is to generate for the ultrasonic horn 8 is executed at a time that is between when the ultrasonic horn 8 and the anvil 14 have unclamped the continuous web 30 in the ultrasonic-sealing processing and when the ultrasonic wave generator 9 starts to generate ultrasonic waves for the ultrasonic horn 8 for the next instance of ultrasonic-sealing processing performed by the ultrasonic horn 8.

For this reason, the frequency adjustment processing is executed in a state where residual vibration has stopped. Accordingly, frequency adjustment can be performed appropriately.

### Other embodiments

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof.

Also, in the above embodiment, a different ultrasonic wave generator 9 generates ultrasonic waves for each of the sealing units 10. In other words, one ultrasonic wave generator 9 is provided for each of the sealing units 10, but there is no limitation to this. For example, instead of the ultrasonic wave generators 9 and the sealing units 10 being provided in one-to-one correspondence, one ultrasonic wave generator 9 may be provided in correspondence with multiple sealing units 10.

In the case of providing one ultrasonic wave generator 9 for each of the sealing units 10, one ultrasonic wave generator 9 is not required to generate ultrasonic waves for multiple sealing units 10. In other words, the ultrasonic wave generator 9 does not need to perform reading/writing/switching a memory for each one of multiple corresponding sealing units 10 (each time the corresponding sealing unit 10 changes) . Also, time is not required for such reading/writing/switching, and the ultrasonic wave generator 9 need only generate ultrasonic waves for one sealing unit 10, and therefore even if the above-described operation mode is the high speed mode, ultrasonic wave generation can be performed without any problem (with sufficient allowance). The above embodiment is desirable in this respect.

Also, in the above embodiment, when ultrasonic-sealing processing for forming a seal portion S is executed, the ultrasonic wave generator 9 starts to generate ultrasonic waves, and then the ultrasonic wave generator 9 stops generating ultrasonic waves when the ultrasonic vibration energy (electric energy) reaches a predetermined amount, but there is no limitation to this. For example, the generation of ultrasonic waves may be stopped after a certain time has elapsed since the ultrasonic wave generator 9 started to generate ultrasonic waves, or when the instantaneous value of the ultrasonic vibration energy of the ultrasonic waves generated by the ultrasonic wave generator 9 (corresponding to the power of the ultrasonic wave generator 9) has exceeded a predetermined threshold value.

Also, in the above embodiment, either the ultrasonic horns 8 or the anvils 14 (first members) are provided at equal intervals on the outer circumferential surface 5A, and the other ones (second members) are provided on the rotation member in correspondence with the first members and can move toward and away from the first members on the opposite side of the continuous web, and in the above embodiment, the first members are the ultrasonic horns 8 and the second members are the anvils 14. However, there is no limitation to this. The first members may be the anvils 14, and the second members may be the ultrasonic horns 8.

Also, although the sealing device 1 is provided with six sealing units 10 in the above embodiment, there is no limitation to this. The sealing device 1 may be provided with seven or more sealing units 10, or five or fewer sealing units 10.

Also, although a link mechanism that employs a cam is described as the swing drive portion in the above embodiment, there is no limitation to this. The operations of the ultrasonic horn 8 and the anvil 14 in the above embodiment may be realized with use of a cylinder mechanism or the like instead of a link mechanism that employs a cam.

Also, although an air damper is used as the first elastic member 71 and the second elastic member 72 in the above embodiment, there is no limitation to this. A coil spring or the like may be used instead of an air damper.

### [Reference Signs List]

1 sealing device, 2 timing wheel, 3 bearing portion, 3a rotation shaft
3b ball bearing, 4 fixed table, 5 rotating drum, 5A outer circumferential surface
5a rotating drum window, 6 rotation base, 7a converter, 7b booster
8 ultrasonic horn, 8a horn facing surface, 9 ultrasonic wave generator,
10 sealing unit
14 anvil, 14B anvil projection portion, 14a anvil facing surface
21 supply transporting roll, 22 discharge transporting roll, 30 continuous web
30A one side of continuous web, 30B other side of continuous web, 31 second sheet
32 first sheet, 32a side edge on one side of first sheet
32b side edge on other side of first sheet, 33 liquid-absorbent body,
34 leg hole
35 first waist band, 36 second waist band, 37 first leg band
38 second leg band, 50 swinging support member, 51 swing portion, 51A swing shaft
51B first coupling shaft, 52 holding portion, 52B attachment surface,
52a frame portion, 52c inward surface
53 drive link, 54 rotation link, 54A rotation link base portion
54B rotation link arm portion, 54a support shaft, 55 second coupling shaft,
56 drive member
56a drive support body, 56b follower, 60 cam member, 61 cam groove
70 support portion, 71 first elastic member, 71A first fixing plate, 71B second fixing plate
72 second elastic member, 73 first nozzle, 74 second nozzle, 75 first air pipe
76 second air pipe, S seal portion, D1 before-vibration-start period,
D2 electrical vibration period
D3 residual vibration period, D4 after-vibration-stop period

## Claims

1. A method for ultrasonic-sealing in which a plurality of seal portions are formed in a continuous web (30) at an interval in a direction of transporting the continuous web (30), the forming being performed by repeatedly executing ultrasonic-sealing processing on a continuous web (30) that is being transported, the continuous web (30) being for an absorbent article, the ultrasonic-sealing processing comprising: a clamping step of clamping the continuous web (30) with an ultrasonic horn (8) and an anvil (14); a seal-portion forming step of forming the seal portions in the continuous web (30) by vibration of the ultrasonic horn (8) due to receiving an ultrasonic wave generated by an ultrasonic wave generator (9); and an unclamping step of unclamping the continuous web (30) after the ultrasonic wave generator (9) has stopped generating the ultrasonic wave and furthermore residual vibration remaining in the ultrasonic horn (8) has stopped

2. The method for ultrasonic-sealing according to claim 1, wherein a period of time from when the residual vibration stops until when the continuous web (30) is unclamped is longer than a period of time from when the ultrasonic wave generator (9) stops generating the ultrasonic wave until when the residual vibration stops.

3. The method for ultrasonic-sealing according to claim 1 or 2, wherein a period of time from when the residual vibration stops until when the continuous web (30) is unclamped is longer than a period of time from when the ultrasonic horn (8) and the anvil (14) start to clamp the continuous web (30) until when the ultrasonic wave generator (9) starts to generate the ultrasonic wave. Z

4. The method for ultrasonic-sealing according to any of claims 1 to 3, wherein
a period of time from when the residual vibration stops until when the continuous web (30) is unclamped
is shorter than
a period of time from when the ultrasonic wave generator (9) starts generating the ultrasonic wave until when the ultrasonic wave generator (9) stops generating the ultrasonic wave.

5. The method for ultrasonic-sealing according to any of claims 1 to 4, wherein
in the clamping step, the continuous web (30) is clamped in a state where the ultrasonic horn (8) is not vibrating.

6. The method for ultrasonic-sealing according to any of claims 1 to 5, wherein
in the seal-portion forming step, after the ultrasonic wave generator (9) starts to generate the ultrasonic wave, the ultrasonic wave generator (9) stops generating the ultrasonic wave when an ultrasonic vibration energy has reached a predetermined amount.

7. The method for ultrasonic-sealing according to any of claims 1 to 6, wherein
in the clamping step, the continuous web (30) is clamped by a flat surface of the ultrasonic horn (8) and a flat surface of the anvil (14).

8. The method for ultrasonic-sealing according to any of claims 1 to 7, wherein
the continuous web (30) is transported by rotation of the rotation member with being wound on an outer circumferential surface of a rotation member,
a plurality of first members, which are either of the ultrasonic horns (8) and the anvils (14), are provided on the outer circumferential surface of the rotation member at an equal interval,
a plurality of second members, which are other ones out of the ultrasonic horns (8) and the anvils (14), are provided on the rotation member in correspondence with the first members,
each of the plurality of second members can move toward and away from the corresponding first member on an opposite side of the continuous web (30),
when each of the ultrasonic horns (8) and the corresponding anvil (14) reaches a predetermined first angular position while rotating along with rotation of the rotation member,
the second member comes into contact with the corresponding first member and clamps the continuous web (30),
when each of the ultrasonic horns (8) and the corresponding anvil (14) reaches a predetermined second angular position while rotating along with rotation of the rotation member,
the second member moves away from the corresponding first member and unclamps the continuous web (30), and
when each of the ultrasonic horns (8) and the corresponding anvil (14) reaches a predetermined third angular position while rotating along with rotation of the rotation member,
the ultrasonic wave generator (9) starts to generate the ultrasonic wave.

9. The method for ultrasonic-sealing according to claim 8, wherein
letting θ be a difference between a third angle and a second angle, and T be a period of time from when the ultrasonic wave generator (9) starts to generate the ultrasonic wave until when the residual vibration stops,
the continuous web (30) is transported by rotation of the rotation member at an angular velocity less than or equal to θ/T.

10. The method for ultrasonic-sealing according to claim 9, wherein
letting a low speed mode be a mode in which the continuous web (30) is transported by rotation of the rotation member at a first speed, and
letting a high speed mode be a mode in which the continuous web (30) is transported by rotation of the rotation member at a second speed that is higher than the first speed,
concerning a period of time from when the residual vibration stops until when the continuous web (30) is unclamped,
the period of time in a case where the plurality of seal portions have been formed in the low speed mode, is longer than the period of time in a case where the plurality of seal portions have been formed in the high speed mode.

11. The method for ultrasonic-sealing according to any of claims 8 to 10, wherein each of the plurality of second members moves toward and away from the corresponding first member by swinging.

12. The method for ultrasonic-sealing according to any of claims 1 to 11, wherein the method for ultrasonic-sealing further comprises a frequency adjustment processing of adjusting a frequency of the ultrasonic wave that the ultrasonic wave generator (9) is to generate for the ultrasonic horn (8), and the frequency adjustment processing is executed at a time that is between when the ultrasonic horn (8) and the anvil (14) have unclamped the continuous web (30) in the ultrasonic-sealing processing and when the ultrasonic wave generator (9) starts to generate the ultrasonic wave for the ultrasonic horn (8) for a next instance of the ultrasonic-sealing processing to be performed by the ultrasonic horn (8).

13. A device for ultrasonic-sealing that forms a plurality of seal portions in a continuous web (30) that is for an absorbent article, the device for ultrasonic-sealing comprising: a transporting device configured to transport the continuous web (30); an ultrasonic wave generator (9) configured to generate an ultrasonic wave; an ultrasonic horn (8) configured to form the seal portion in the continuous web (30) that is being transported by the transporting device, by vibrating due to receiving the ultrasonic wave generated by the ultrasonic wave generator (9); and an anvil (14) configured to clamp the continuous web (30) together with the ultrasonic horn (8), the device being configured in that the ultrasonic horn (8) forms the plurality of seal portions at an interval in a direction of transporting the continuous web (30) by repeatedly executing ultrasonic-sealing processing, the ultrasonic-sealing processing being for forming a seal portion in the continuous web (30) by vibrating while clamping the continuous web (30) together with the anvil (14), the ultrasonic horn (8) and the anvil (14) unclamping the continuous web (30) after the ultrasonic wave generator (9) has stopped generating the ultrasonic wave and furthermore residual vibration remaining in the ultrasonic horn (8) has stopped.

## Patentansprüche

1. Verfahren zum Ultraschallsiegeln, bei dem eine Mehrzahl von Siegelnahtabschnitten in einer Endlosbahn (30) in einem Abstand in einer Transportrichtung der Endlosbahn (30) ausgebildet wird, wobei das Ausbilden durch wiederholtes Ausführen eines Ultraschallsiegelvorgangs an einer Endlosbahn (30), die transportiert wird, durchgeführt wird, wobei die Endlosbahn (30) für einen absorbierenden Artikel vorgesehen ist, wobei der Ultraschallsiegelvorgang umfasst: einen Einspannschritt des Einspannens der Endlosbahn (30) mit einem Ultraschallhorn (8) und einem Amboss (14); einen Siegelnahtabschnitt-Ausbildungsschritt des Ausbildens der Siegelnahtabschnitte in der Endlosbahn (30) durch Vibration des Ultraschallhorns (8) aufgrund des Empfangens einer von einem Ultraschallwellengenerator (9) erzeugten Ultraschallwelle; und einen Ausspannungsschritt des Ausspannens der Endlosbahn (30), nachdem der Ultraschallwellengenerator (9) das Erzeugen der Ultraschallwelle gestoppt hat und außerdem die in dem Ultraschallhorn (8) verbleibende Restvibration gestoppt hat.

2. Verfahren zum Ultraschallsiegeln nach Anspruch 1, wobei eine Zeitspanne ab dem Zeitpunkt, an dem die Restvibration stoppt, bis zu dem Zeitpunkt, zu dem die Endlosbahn (30) ausgespannt wird, länger ist als eine Zeitspanne ab dem Zeitpunkt, zu dem der Ultraschallwellengenerator (9) das Erzeugen der Ultraschallwelle stoppt, bis zu dem Zeitpunkt, an dem die Restschwingung stoppt.

3. Verfahren zum Ultraschallsiegeln nach Anspruch 1 oder 2, wobei eine Zeitspanne ab dem Zeitpunkt, zu dem die Restschwingung stoppt, bis zu dem Zeitpunkt, zu dem die Endlosbahn (30) ausgespannt wird, länger ist als eine Zeitspanne ab dem Zeitpunkt, zu dem das Ultraschallhorn (8) und der Amboss (14) beginnen, die Endlosbahn (30) einzuspannen, bis zu dem Zeitpunkt, zu dem der Ultraschallwellengenerator (9) beginnt, die Ultraschallwelle zu erzeugen.

4. Verfahren zum Ultraschallsiegeln nach einem der Ansprüche 1 bis 3, wobei eine Zeitspanne ab dem Zeitpunkt, zu dem die Restschwingung stoppt, bis zu dem Zeitpunkt, an dem die Endlosbahn (30) ausgespannt wird, kürzer ist als eine Zeitspanne ab dem Zeitpunkt, zu dem der Ultraschallwellengenerator (9) mit dem Erzeugen der Ultraschallwelle beginnt, bis zu dem Zeitpunkt, zu dem der Ultraschallwellengenerator (9) das Erzeugen der Ultraschallwelle stoppt.

5. Verfahren zum Ultraschallsiegeln nach einem der Ansprüche 1 bis 4, wobei in dem Einspannschritt die Endlosbahn (30) in einem Zustand eingespannt wird, in dem das Ultraschallhorn (8) nicht vibriert.

6. Verfahren zum Ultraschallsiegeln nach einem der Ansprüche 1 bis 5, wobei in dem Siegelnahtabschnitt-Ausbildungsschritt, nachdem der Ultraschallwellengenerator (9) beginnt, die Ultraschallwelle zu erzeugen, der Ultraschallwellengenerator (9) das Erzeugen der Ultraschallwelle stoppt, wenn eine Ultraschallvibrationsenergie einen vorbestimmten Betrag erreicht hat.

7. Verfahren zum Ultraschallsiegeln nach einem der Ansprüche 1 bis 6, wobei in dem Einspannschritt die Endlosbahn (30) durch eine ebene Fläche des Ultraschallhorns (8) und eine ebene Fläche des Ambosses (14) eingespannt wird.

8. Verfahren zum Ultraschallsiegeln nach einem der Ansprüche 1 bis 7, wobei
die Endlosbahn (30) durch Rotation des Rotationselements transportiert wird, indem sie auf eine Außenumfangsfläche eines Rotationselements gewickelt wird,
eine Mehrzahl von ersten Elementen, die entweder die Ultraschallhörner (8) oder die Ambosse (14) sind, in einem gleichen Abstand auf der Außenumfangsfläche des Rotationselements vorgesehen sind,
eine Mehrzahl von zweiten Elementen, die die anderen von den Ultraschallhörnern (8) und den Ambossen (14) sind, in Entsprechung zu den ersten Elementen auf dem Rotationselement vorgesehen sind,
jedes von der Mehrzahl von zweiten Elementen sich zu dem entsprechenden ersten Element auf einer gegenüberliegenden Seite der Endlosbahn (30) hin und von diesem weg bewegen kann,
wenn jedes der Ultraschallhörner (8) und der entsprechende Amboss (14) eine vorbestimmte erste Winkelposition erreichen, während sie sich zusammen mit der Drehung des Rotationselements drehen,
das zweite Element mit dem entsprechenden ersten Element in Kontakt kommt und die Endlosbahn (30) einspannt,
wenn jedes der Ultraschallhörner (8) und der entsprechende Amboss (14) eine vorbestimmte zweite Winkelposition erreichen, während sie sich zusammen mit der Rotation des Rotationselements drehen,
das zweite Element sich von dem entsprechenden ersten Element wegbewegt und die Endlosbahn (30) ausspannt, und
wenn jedes der Ultraschallhörner (8) und der entsprechende Amboss (14) eine vorbestimmte dritte Winkelposition erreichen, während sie sich zusammen mit der Rotation des Rotationselements drehen,
der Ultraschallwellengenerator (9) beginnt, die Ultraschallwelle zu erzeugen.

9. Verfahren zum Ultraschallsiegeln nach Anspruch 8, wobei
wenn θ eine Differenz sei zwischen einem dritten Winkel und einem zweiten Winkel, und T eine Zeitspanne sei ab dem Zeitpunkt, zu dem der Ultraschallwellengenerator (9) beginnt, die Ultraschallwelle zu erzeugen, bis zu dem Zeitpunkt, zu dem die Restschwingung stoppt,
die Endlosbahn (30) durch Rotation des Rotationselements mit einer Winkelgeschwindigkeit kleiner oder gleich θ/T transportiert wird.

10. Verfahren zum Ultraschallsiegeln nach Anspruch 9, wobei
ein Niedriggeschwindigkeitsmodus ein Modus sei, in dem die Endlosbahn (30) durch Rotation des Rotationselements mit einer ersten Geschwindigkeit transportiert wird, und
ein Hochgeschwindigkeitsmodus ein Modus sei, in dem die Endlosbahn (30) durch Rotation des Rotationselements mit einer zweiten Geschwindigkeit transportiert wird, die höher ist als die erste Geschwindigkeit,
dann ist, bezüglich einer Zeitspanne ab dem Zeitpunkt, zu dem die Restschwingung stoppt, bis zu dem Zeitpunkt, zu dem die Endlosbahn (30) ausgespannt wird,
die Zeitspanne in einem Fall, in dem die Mehrzahl von Siegelnahtabschnitten in dem Niedriggeschwindigkeitsmodus ausgebildet worden ist, länger als die Zeitspanne in einem Fall, in dem die Mehrzahl von Siegelnahtabschnitten in dem Hochgeschwindigkeitsmodus ausgebildet worden ist.

11. Verfahren zum Ultraschallsiegeln nach einem der Ansprüche 8 bis 10, wobei sich jedes der Mehrzahl von zweiten Elementen durch Schwingen auf das entsprechende erste Element zu und von diesem weg bewegt.

12. Verfahren zum Ultraschallsiegeln nach einem der Ansprüche 1 bis 11, wobei das Verfahren zum Ultraschallsiegeln ferner einen Frequenzeinstellungsvorgang zum Einstellen einer Frequenz der Ultraschallwelle umfasst, die der Ultraschallwellengenerator (9) für das Ultraschallhorn (8) erzeugen soll, und der Frequenzeinstellungsvorgang zu einem Zeitpunkt ausgeführt wird, der zwischen dem Zeitpunkt, zu dem das Ultraschallhorn (8) und der Amboss (14) die Endlosbahn (30) bei dem Ultraschallsiegelungsvorgang ausgespannt haben, und dem Zeitpunkt liegt, zu dem der Ultraschallwellengenerator (9) beginnt, die Ultraschallwelle für das Ultraschallhorn (8) für eine nächste Instanz des von dem Ultraschallhorn (8) auszuführenden Ultraschallsiegelungsvorgangs zu erzeugen.

13. Vorrichtung zum Ultraschallsiegeln, die eine Mehrzahl von Siegelnahtabschnitten in einer Endlosbahn (30) ausbildet, die für einen absorbierenden Artikel vorgesehen ist, wobei die Vorrichtung zum Ultraschallsiegeln umfasst: eine Transportvorrichtung, die dazu konfiguriert ist, die Endlosbahn (30) zu transportieren; einen Ultraschallwellengenerator (9), der dazu konfiguriert ist, eine Ultraschallwelle zu erzeugen; ein Ultraschallhorn (8), das dazu konfiguriert ist, den Siegelnahtabschnitt in der Endlosbahn (30), die von der Transportvorrichtung transportiert wird, durch Vibration aufgrund des Empfangens der von dem Ultraschallwellengenerator (9) erzeugten Ultraschallwelle auszubilden; und einen Amboss (14), der dazu konfiguriert ist, zusammen mit dem Ultraschallhorn (8) die Endlosbahn (30) einzuspannen, wobei die Vorrichtung so konfiguriert ist, dass das Ultraschallhorn (8) die Mehrzahl von Siegelnahtabschnitten in einem Abstand in einer Transportrichtung der Endlosbahn (30) durch wiederholtes Ausführen eines Ultraschallsiegelungsvorgangs ausbildet, wobei der Ultraschallsiegelungsvorgang zum Ausbilden eines Siegelnahtabschnitts in der Endlosbahn (30) durch Vibrieren dient, während die Endlosbahn (30) zusammen mit dem Amboss (14) eingespannt wird, wobei das Ultraschallhorn (8) und der Amboss (14) die Endlosbahn (30) ausspannen, nachdem der Ultraschallwellengenerator (9) das Erzeugen der Ultraschallwelle gestoppt hat, und außerdem die in dem Ultraschallhorn (8) verbleibende Restvibration gestoppt hat.

## Revendications

1. Procédé de soudage par ultrasons, dans lequel une pluralité de parties de soudure sont formées dans une bande continue (30) à un intervalle dans une direction de transport de la bande continue (30), la formation étant réalisée par exécution répétée d'un traitement de soudage par ultrasons sur une bande continue (30) qui est en train d'être transportée, la bande continue (30) étant destinée à un article absorbant, le traitement de soudage par ultrasons comprenant : une étape de serrage consistant à serrer la bande continue (30) avec une sonotrode (8) et une enclume (14) ; une étape de formation de parties de soudure consistant à former les parties de soudure dans la bande continue (30) par vibration de la sonotrode (8) en raison de la réception d'une onde ultrasonore générée par un générateur d'onde ultrasonore (9) ; et une étape de desserrage consistant à desserrer la bande continue (30) après que le générateur d'onde ultrasonore (9) a arrêté de générer l'onde ultrasonore et, en outre, qu'une vibration résiduelle restant dans la sonotrode (8) s'est arrêtée.

2. Procédé de soudage par ultrasons selon la revendication 1, dans lequel une période de temps allant du moment où la vibration résiduelle s'arrête jusqu'au moment où la bande continue (30) est desserrée, est plus longue qu'une période de temps allant du moment où le générateur d'onde ultrasonore (9) arrête de générer l'onde ultrasonore jusqu'au moment où la vibration résiduelle s'arrête.

3. Procédé de soudage par ultrasons selon la revendication 1 ou 2, dans lequel une période de temps allant du moment où la vibration résiduelle s'arrête jusqu'au moment où la bande continue (30) est desserrée, est plus longue qu'une période de temps allant du moment où la sonotrode (8) et l'enclume (14) commencent à serrer la bande continue (30) jusqu'au moment où le générateur d'onde ultrasonore (9) commence à générer l'onde ultrasonore.

4. Procédé de soudage par ultrasons selon l'une quelconque des revendications 1 à 3, dans lequel une période de temps allant du moment où la vibration résiduelle s'arrête jusqu'au moment où la bande continue (30) est desserrée, est plus courte qu'une période de temps allant du moment où le générateur d'onde ultrasonore (9) commence à générer l'onde ultrasonore jusqu'au moment où le générateur d'onde ultrasonore (9) arrête de générer l'onde ultrasonore.

5. Procédé de soudage par ultrasons selon l'une quelconque des revendications 1 à 4, dans lequel, dans l'étape de serrage, la bande continue (30) est serrée dans un état dans lequel la sonotrode (8) ne vibre pas.

6. Procédé de soudage par ultrasons selon l'une quelconque des revendications 1 à 5, dans lequel, dans l'étape de formation de parties de soudure, après que le générateur d'onde ultrasonore (9) a commencé à générer l'onde ultrasonore, le générateur d'onde ultrasonore (9) arrête de générer l'onde ultrasonore lorsqu'une énergie de vibration ultrasonore a atteint une quantité prédéterminée.

7. Procédé de soudage par ultrasons selon l'une quelconque des revendications 1 à 6, dans lequel, dans l'étape de serrage, la bande continue (30) est serrée par une surface plate de la sonotrode (8) et une surface plate de l'enclume (14).

8. Procédé de soudage par ultrasons selon l'une quelconque des revendications 1 à 7, dans lequel
la bande continue (30) est transportée par rotation de l'élément de rotation en étant enroulée sur une surface circonférentielle externe d'un élément de rotation,
une pluralité de premiers éléments, qui sont soit les sonotrode (8) soit les enclumes (14), sont disposés sur la surface circonférentielle externe de l'élément de rotation à intervalle égal,
une pluralité de seconds éléments, qui sont les autres parmi les sonotrode (8) et les enclumes (14), sont disposés sur l'élément de rotation en correspondance avec les premiers éléments,
chacun parmi la pluralité de seconds éléments peut se déplacer vers et à l'opposé du premier élément correspondant sur un côté opposé de la bande continue (30),
lorsque chacune des sonotrodes (8) et l'enclume correspondante (14) atteignent une première position angulaire prédéterminée tout en tournant conjointement avec la rotation de l'élément de rotation,
le second élément entre en contact avec le premier élément correspondant et serre la bande continue (30),
lorsque chacune des sonotrodes (8) et l'enclume correspondante (14) atteignent une deuxième position angulaire prédéterminée tout en tournant conjointement avec la rotation de l'élément de rotation,
le second élément se déplace à l'opposé du premier élément correspondant et desserre la bande continue (30), et
lorsque chacune des sonotrodes (8) et l'enclume correspondante (14) atteignent une troisième position angulaire prédéterminée tout en tournant conjointement avec la rotation de l'élément de rotation,
le générateur d'onde ultrasonore (9) commence à générer l'onde ultrasonore.

9. Procédé de soudage par ultrasons selon la revendication 8, dans lequel,
θ étant une différence entre un troisième angle et un deuxième angle, et T étant une période de temps allant du moment où le générateur d'onde ultrasonore (9) commence à générer l'onde ultrasonore jusqu'au moment où la vibration résiduelle s'arrête,
la bande continue (30) est transportée par rotation de l'élément de rotation à une vitesse angulaire inférieure ou égale à θ/T.

10. Procédé de soudage par ultrasons selon la revendication 9, dans lequel,
un mode à faible vitesse étant un mode dans lequel la bande continue (30) est transportée par rotation de l'élément de rotation à une première vitesse, et
un mode à haute vitesse étant un mode dans lequel la bande continue (30) est transportée par rotation de l'élément de rotation à une seconde vitesse qui est plus élevée que la première vitesse,
concernant une période de temps allant du moment où la vibration résiduelle s'arrête jusqu'au moment où la bande continue (30) est desserrée,
la période de temps dans un cas où la pluralité de parties de soudure ont été formées dans le mode à faible vitesse est plus longue que la période de temps dans un cas où la pluralité de parties de soudure ont été formées dans le mode à haute vitesse.

11. Procédé de soudage par ultrasons selon l'une quelconque des revendications 8 à 10, dans lequel chacun parmi la pluralité de seconds éléments se déplace vers et à l'opposé du premier élément correspondant par balancement.

12. Procédé de soudage par ultrasons selon l'une quelconque des revendications 1 à 11, dans lequel le procédé de soudage par ultrasons comprend en outre un traitement d'ajustement de fréquence consistant à ajuster une fréquence de l'onde ultrasonore que le générateur d'onde ultrasonore (9) doit générer pour la sonotrode (8), et le traitement d'ajustement de fréquence est réalisé à un moment qui se situe entre le moment où la sonotrode (8) et l'enclume (14) ont desserré la bande continue (30) dans le traitement de soudage par ultrasons et le moment où le générateur d'onde ultrasonore (9) commence à générer l'onde ultrasonore pour la sonotrode (8) pour une occurrence suivante du traitement de soudage par ultrasons devant être réalisé par la sonotrode (8).

13. Dispositif pour soudage par ultrasons qui forme une pluralité de parties de soudure dans une bande continue (30) qui est destinée à un article absorbant, le dispositif pour soudage par ultrasons comprenant : un dispositif de transport configuré pour transporter la bande continue (30) ; un générateur d'onde ultrasonore (9) configuré pour générer une onde ultrasonore ; une sonotrode (8) configurée pour former la partie de soudure dans la bande continue (30) qui est en train d'être transportée par le dispositif de transport, par vibration en raison de la réception de l'onde ultrasonore générée par le générateur d'onde ultrasonore (9) ; et une enclume (14) configurée pour serrer la bande continue (30) conjointement avec la sonotrode (8), le dispositif étant configuré de telle sorte que la sonotrode (8) forme la pluralité de parties de soudure à un intervalle dans une direction de transport de la bande continue (30) par exécution répétée d'un traitement de soudage par ultrasons, le traitement de soudage par ultrasons servant à former une partie de soudure dans la bande continue (30) par vibration tout en serrant la bande continue (30) conjointement avec l'enclume (14), la sonotrode (8) et l'enclume (14) desserrant la bande continue (30) après que le générateur d'onde ultrasonore (9) a arrêté de générer l'onde ultrasonore et, en outre, qu'une vibration résiduelle restant dans la sonotrode (8) s'est arrêtée.
